# EUROPEAN PATENT APPLICATION

(11) **EP 3 345 533 A2**
(43) Date of publication of application: **11.07.2018**
(21) Application number: 16842229.3
(22) Date of filing: 29.08.2016
(51) Int. Cl.: A61B 5/00, H04L 12/28

(54) **SLEEP MANAGEMENT METHOD**

(30) Priority: 03.09.2015 KR 20150124569; 19.08.2016 KR 20160105286
(71) Applicant: Samsung Electronics Co., Ltd., Suwon-si, Gyeonggi-do 16677 (KR)
(72) Inventor: PARK, Gi Eun, Anyang-si Gyeonggi-do 14027 (KR); LIM, Kyoung Ae, Seoul 06288 (KR); KIM, Ji Kyoung, Yongin-si Gyeonggi-do 16846 (KR); YOON, Ji Yoon, Seoul 06241 (KR); SHIN, Dong Hyuk, Yongin-si Gyeonggi-do 16953 (KR); SHIN, Young Sun, Seongnam-si Gyeonggi-do 13611 (KR); LEE, Jin Young, Suwon-si Gyeonggi-do 16488 (KR)
(74) Representative: Gulde & Partner
(86) International application number: PCT/KR2016/009593
(87) International publication number: WO 2017/039263

(57) **Abstract**

Disclosed herein are a sleep management method includes obtaining sleep data of a user, obtaining sleep information of the user on the basis of the sleep data of the user, identifying a user sleep type group to which a sleep type of the user belongs among a plurality of sleep type groups on the basis of the sleep information of the user, and displaying sleep advice corresponding to the user sleep type group.

## Description

### [Technical Field]

The present disclosure is a sleep management method, and more particularly, to a sleep management method that obtains sleep information of a user in a non-contact manner.

### [Background Art]

Generally, home network systems detect a state or operation of a user and control operation of various electronic devices depending on the state or operation of the user.

To this end, electronic devices of home network systems generally include various sensors.

For example, some electronic devices may obtain a facial image of a user by using a camera, process the user's facial image, and estimate an emotion of the user.

Also, other electronic devices may obtain a movement of the user by using an infrared sensor module and then operate depending on the user's movement.

In particular, recently, research is being actively conducted on sensor technology for detecting whether a user is sleeping.

### [Disclosure]

### [Technical Problem]

An aspect of the present disclosure is directed to providing a sleep management method capable of determining a sleep state of a user.

Another aspect of the present disclosure is directed to providing a sleep management method capable of providing sleep information of a user to the user.

Still another aspect of the present disclosure is directed to providing a sleep management method capable of sharing sleep information of a user with a predetermined sharer.

### [Technical Solution]

In accordance with one aspect of the present disclosure, a sleep management method includes obtaining sleep data of a user, obtaining sleep information of the user on the basis of the sleep data of the user, identifying a user sleep type group to which a sleep type of the user belongs among a plurality of sleep type groups on the basis of the sleep information of the user, and displaying sleep advice corresponding to the user sleep type group.

The sleep management method may further include calculating a sleep score of the user on the basis of the sleep information of the user.

The sleep management method may further include displaying both the sleep score of the user and an average sleep score of an age group in which the user is included.

The sleep management method may further include displaying the sleep information of the user.

The sleep management method may further include receiving physical information of the user from the user, and the physical information may include at least one of a gender, height, and weight of the user.

The sleep management method may further include displaying sleep advice corresponding to the physical information of the user.

The sleep management method may further include receiving activity information of the user from the user, and the activity information may include at least one of whether the user drinks, whether the user takes a stimulant, and whether the user exercises.

The sleep management method may further include displaying sleep advice corresponding to the activity information of the user.

The sleep management method may further include receiving living information of the user from at least one electronic device, and the living information may include at least one of a user input received by the electronic device from the user and detection data detected by the electronic device from the user.

The sleep management method may further include displaying sleep advice corresponding to the living information of the user.

The sleep management method may further include transmitting sleep advice corresponding to the living information of the user to the at least one electronic device.

The sleep management method may further include sharing the sleep information and the sleep advice with a sharer set by the user.

The sharing of the sleep information and the sleep advice may include receiving at least one of identification information of the sharer and identification information of a user terminal of the sharer, and transmitting the sleep information and the sleep advice to the user on the basis of the identification information of the sharer and the identification information of the user terminal of the sharer.

The sharing of the sleep information and the sleep advice with a sharer may include receiving the sleep information and a sharing time of the sleep information and the sleep advice from the user, and transmitting the sleep information and the sleep advice to the sharer at the sharing time.

In accordance with one aspect of the present disclosure, a sleep management method includes obtaining sleep data of a user, identifying sleep state of the user on the basis of the sleep data of the user, identifying a sleep abnormality of the user on the basis of the sleep data of the user and the sleep state of the user, and transmitting a sleep abnormality message of the user in response to identifying of the sleep abnormality of the user.

The identifying of the sleep abnormality of the user may include identifying a heart rate abnormality of the user on the basis of a heart rate of the user included in the sleep data of the user.

The identifying of the sleep abnormality of the user may include identifying a respiratory disturbance or a non-respiration of the user on the basis of the number of breaths of the user included in the sleep data of the user.

The transmitting of the sleep abnormality message of the user may include transmitting the sleep abnormality message of the user to a sharer set by the user.

The transmitting of the sleep abnormality message of the user may include transmitting the sleep abnormality message of the user to a previously set electronic device.

The sleep management method may further include providing a wake-up alarm to the user when the sleep state is a REM sleep stage and a remaining time until a waking-up time set by the user is less than a reference time.

### [Advantageous Effects]

According to an aspect of the present disclosure, it is possible to provide a sleep management method capable of determining a sleep state of a user.

According to another aspect of the present disclosure, it is possible to provide a sleep management method capable of providing sleep information of a user to the user.

According to still another aspect of the present disclosure, it is possible to provide a sleep management method capable of sharing sleep information of a user with a predetermined sharer.

### [Description of Drawings]

FIG. 1 shows an example of a configuration of a sleep management system according to an embodiment.
FIG. 2 shows another example of a configuration of a sleep management system according to an embodiment.
FIG. 3 shows an example of operation of a sleep management system according to an embodiment.
FIG. 4 shows an example of a user's sleep cycle shown in FIG. 3.
FIG. 5 shows a configuration of a sleep data acquisition apparatus included in a sleep management system according to an embodiment.
FIG. 6 shows a configuration of a user terminal included in a sleep management system according to an embodiment.
FIG. 7 shows an example of a sleep information display operation of a sleep management system according to an embodiment.
FIG. 8 shows an example of a summary sleep information screen displayed by a sleep management system according to an embodiment.
FIG. 9 shows an example of a daily sleep information screen displayed by a sleep management system according to an embodiment.
FIG. 10 shows an example of a monthly sleep information screen displayed by a sleep management system according to an embodiment.
FIG. 11 shows an example of a sleep advice screen displayed by a sleep management system according to an embodiment.
FIG. 12 shows another example of a sleep information display operation of a sleep management system according to an embodiment.
FIG. 13 shows an example of a screen in which a sleep management system obtains physical information of a user according to an embodiment.
FIG. 14 shows an example of a screen in which a sleep management system obtains sleep-associated activity information of a user according to an embodiment.
FIG. 15 shows an example of a sleep information sharing operation of a sleep management system according to an embodiment.
FIG. 16 shows an example of a screen in which a sleep management system obtains settings about whether to share sleep information according to an embodiment.
FIG. 17 shows an example in which a sleep management system displays sleep information shared with a third party according to an embodiment.
FIG. 18 shows an example of an interoperation with an electronic device of a sleep management system according to an embodiment.
FIG. 19 shows an example in which a sleep management system collects data from an electronic device according to an embodiment.
FIG. 20 shows an example in which a sleep management system provides sleep information to an electronic device according to an embodiment.
FIG. 21 shows an example of a sleep abnormality notification operation of a sleep management system according to an embodiment.
FIG. 22 shows an example of a screen in which a sleep management system obtains a setting of whether to perform notification of an abnormality according to an embodiment
FIG. 23 shows an example in which a sleep management system transmits a sleep abnormality notification to an electronic device according to an embodiment.
FIG. 24 shows an example of an alarm operation of a sleep management system according to an embodiment.
FIG. 25 shows a wake-up time setting screen for an alarm operation of a sleep management system according to an embodiment.
FIG. 26 shows a sleep pattern of a user for illustrating an alarm operation of a sleep management system according to an embodiment.
FIG. 27 shows an example of a sleep inducing operation of a sleep management system according to an embodiment.

### [Best Mode]

The configurations shown in the embodiments and drawings described herein are merely examples of preferred embodiments of the present disclosure, and there may be various modifications at the time of filing of the present application that replace the embodiments and drawings.

The terminology used herein is for the purpose of describing particular embodiments only and is not intended to limit the scope of the present disclosure.

Specifically, the singular forms "a," "an," and "the" include the plural forms as well, unless the context clearly dictates otherwise.

Also, the terms "comprises," "includes," or "has," when used herein, should be understood as specifying the presence of stated features, integers, steps, operations, elements, components, or combinations thereof and not precluding the presence or addition of one or more other features, integers, steps, operations, elements, components, or combinations thereof.

In addition, the terms including ordinals such as "first," "second," and the like used in the present specification may be used to describe various elements, but these elements are not limited by the terms. These terms are used to only distinguish one element from another element.

Also, terms such as "unit," "-er," "-or," "block," "member," "module," and the like used in the present specification may represent a unit which processes at least one function or operation. For example, the terms may represent hardware such as a field-programmable gate array (FPGA) or an application specific integrated circuit (ASIC), software stored in a memory, or one or more processes processed by a processor.

Hereinafter, embodiments of the present disclosure will be described in detail with reference to the accompanying drawings. The same reference numeral or symbol shown in the accompanying drawings may represent a part or an element for performing substantially the same function.

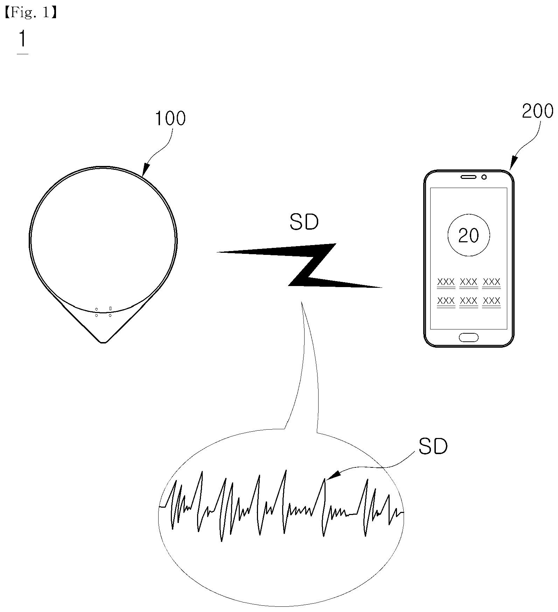

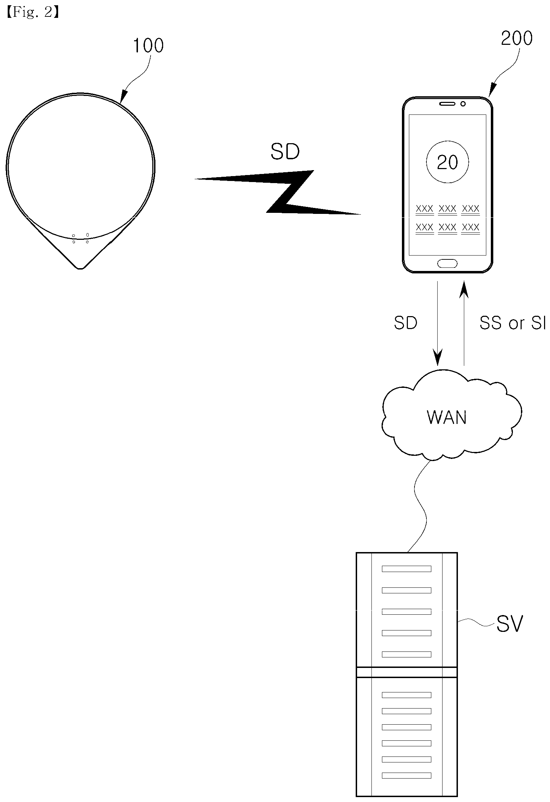

FIG. 1 shows an example of a configuration of a sleep management system according to an embodiment, and FIG. 2 shows another example of a configuration of a sleep management system according to an embodiment.

As shown in FIG. 1, a sleep management system 1 according to an embodiment may include a sleep data acquisition apparatus 100 configured to obtain sleep-related data SD (hereinafter referred to as sleep data) of a user and a user terminal 200 configured to obtain sleep-related information (hereinafter referred to as sleep information) of the user on the basis of the sleep data SD. In this case, the sleep information may include sleep time information related to a sleep time of a user U, and sleep summary information to be displayed to the user U.

The sleep data acquisition apparatus 100 may collect the sleep data SD of the user while the user is sleeping, falling asleep, or waking up. Also, the sleep data acquisition apparatus 100 may transmit the sleep data SD of the user to the user terminal 200.

In detail, the sleep data acquisition apparatus 100 may detect a heart rate, respiration rate, and movement of the user and generate sleep data SD corresponding to the user's heart rate, respiration rate, and movement. For example, the sleep data acquisition apparatus 100 may detect a vibration caused by the user's heart rate, respiration rate, and movement and generate sleep data SD corresponding to the detected vibration.

Also, the sleep data acquisition apparatus 100 may transmit pieces of sleep data SD generated every predetermined time interval to the user terminal 200 in a wireless manner.

The user terminal 200 may receive the sleep data SD of the user from the sleep data acquisition apparatus 100, and may obtain a sleep state SS of the user or sleep information SI of the user on the basis of the received sleep data SD.

For example, the user terminal 200 may obtain the sleep state SS of the user U. In detail, the user terminal 200 may determine whether the user U is lying on a bed B, whether the user U falls asleep, in which sleep stage the user U is, whether the user U wakes up briefly while sleeping, whether the user U falls asleep again, whether the user U completely wakes up, whether the user U gets out of the bed B, and the like.

Also, the user terminal 200 may obtain sleep time information such as a time at which the user U lies on the bed B, a time at which the user U falls asleep, a time at which the sleep stage of the user U changes, a time at which the user U wakes up briefly while sleeping, a time at which the user U falls asleep again, a time at which the user U wakes up, and a time at which the user U gets out of the bed B on the basis of the sleep state SS of the user U.

Also, the user terminal 200 may process the sleep time information of the user U, generate sleep summary information, and display the sleep summary information to the user U. For example, the user terminal 200 may display a total sleep time of the user, a time it takes the user to fall asleep, the number of times the user wakes up while sleeping, sleep efficiency, a deep sleep time, a rapid eye movement (REM) sleep time, or the like on the basis of the sleep data SD of the user.

However, the configuration of the sleep management system 1 is not limited to that shown in FIG. 1.

For example, as shown in FIG. 2, the sleep management system 1 may further include a sleep management server SV in addition to the sleep data acquisition apparatus 100 and the user terminal 200.

The sleep management server SV may receive the sleep data SD from the user terminal 200, process the received sleep data SD, and generate the sleep state SS or the sleep information SI of the user U. Also, the sleep management server SV may transmit the sleep state SS or the sleep information SI of the user U to the user terminal 200.

In other words, when the sleep data SD is obtained from the sleep data acquisition apparatus 100, the user terminal 200 may transmit the obtained sleep data SD to the sleep management server SV over a wide area network (WAN) and receive the sleep state SS or the sleep information SI of the user U from the sleep management server SV. Also, when the sleep state SS of the user U is received from the sleep management server SV, the user terminal 200 may generate the sleep information SI of the user U on the basis of the sleep state SS of the user U.

In particular, the sleep management server SV may obtain sleep data of a plurality of users from a plurality of user terminals, process the sleep data, and generate sleep information of the plurality of users. In this case, the sleep management server SV may manage the sleep information of the plurality of users on a user basis.

In addition, the sleep management server SV may manage the sleep information SI of the plurality of users on a group basis, wherein the groups are set by the users. For example, when the plurality of users are set as family members, the sleep management server SV may transmit sleep information of the family members (the plurality of users) to user terminals of the family members so that the family members can share the sleep information.

Operation of the sleep management system 1 will be described in detail below with reference to the following example.

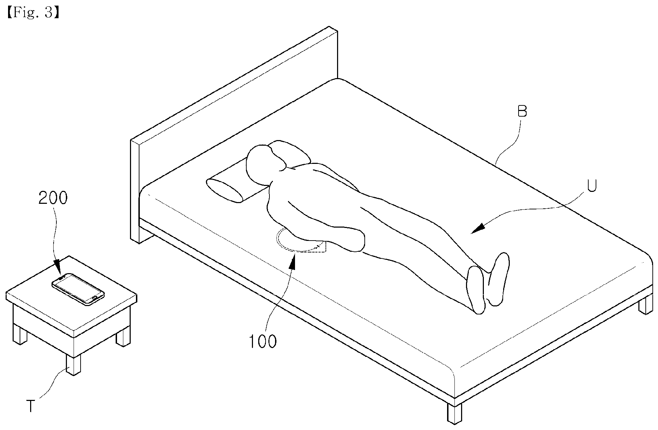

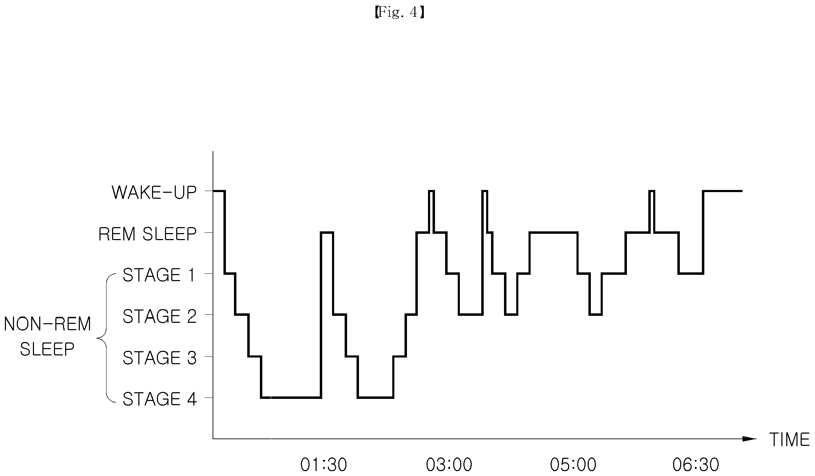

FIG. 3 shows an example of operation of a sleep management system according to an embodiment, and FIG. 4 shows an example of a user's sleep cycle shown in FIG. 3.

As shown in FIG. 3, the sleep data acquisition apparatus 100 may be placed near a user U who is lying on a bed B. For example, as shown in FIG. 3, the sleep data acquisition apparatus 100 may be placed under the bed B of the user U. However, the placement of the sleep data acquisition apparatus 100 is not limited to that shown in FIG. 3, and the sleep data acquisition apparatus 100 may be placed wherever a heart rate, respiration rate, and movement of the user U can be detected.

Also, the user terminal 200 may be placed near the sleep data acquisition apparatus 100. For example, as shown in FIG. 3, the user terminal 200 may be placed on a table T provided near the bed B of the user U. However, the placement of the user terminal 200 is not limited to that shown in FIG. 3, and the user terminal 200 may be placed wherever the sleep data SD can be received from the sleep data acquisition apparatus 100.

As shown in FIG. 3, when the user U is lying on the bed B, the sleep data acquisition apparatus 100 may detect a heart rate, respiration rate, and movement of the user U who is lying on the bed B and transmit sleep data SD corresponding to the user U's heart rate, respiration rate, and movement to the user terminal 200. Also, the user terminal 200 may determine a time at which the user U lies on the bed B on the basis of the sleep data SD or by using the sleep management server SV.

For example, the sleep data acquisition apparatus 100 can detect an amount of vibration less than a reference value, which is caused in daily life of the user U, before the user U lies on the bed B. However, the sleep data acquisition apparatus 100 may interpret the amount of vibration less than the reference value as meaningless noise and may not transmit data corresponding to the amount of vibration less than the reference value to the user terminal 100. As a result, the user terminal 200 may not receive sleep data from the sleep data acquisition apparatus 100 before the user U lies on the bed B.

On the other hand, the user terminal 200 may detect an amount of vibration greater than or equal to the reference value when the user U lies on the bed B. The sleep data acquisition apparatus 100 may transmit sleep data corresponding to the vibration greater than or equal to the reference value to the user terminal 200.

When the sleep data is received from the sleep data acquisition apparatus 100, the user terminal 200 or the sleep management server SV may determine that the user U is lying on the bed B and determine a start time of the reception of the sleep data as a time at which the user U lies on the bed B.

Also, when the user U falls asleep, the sleep data acquisition apparatus 100 may detect a heart rate, respiration rate, and movement of the user U who is sleeping and transmit sleep data SD corresponding to the user U's heart rate, respiration rate, and movement to the user terminal 200. Also, the user terminal 200 may determine a time at which the user U falls asleep on the basis of the sleep data SD or by using the sleep management server SV.

In detail, the user U may not fall asleep as soon as the user U lies on the bed B and may not fall asleep for a short period of several minutes or for several hours. Accordingly, the time at which the user U lies on the bed B may be different from the time at which the user falls asleep.

Also, the user U has a different heart rate, respiration rate, and movement when he or she is awake than when he or she is asleep. In detail, it is well known that a person has a decreased heart rate and a respiration rate while sleeping.

Accordingly, when the sleep data is received from the sleep data acquisition apparatus 100, the user terminal 200 or the sleep management server SV may determine whether the user U is awake or asleep on the basis of the user U's heart rate, respiration rate, and movement and determine the time at which the user falls asleep on the basis of a result of the determination of whether the user U is awake or asleep.

Also, while the user U is sleeping, the sleep data acquisition apparatus 100 may detect the user U's heart rate, respiration rate, and movement every predetermined time interval and transmit sleep data SD corresponding to the user U's heart rate, respiration rate, and movement to the user terminal 200.

On the basis of the sleep data SD of the user U during sleep, the user terminal 200 or the sleep management server SV may determine a sleep stage of the user U and determine whether the user U consciously or unconsciously wakes up.

It is well known that a person does not suddenly fall asleep, maintain deep sleep, and then suddenly wake up, but that human sleep includes certain stages and cycles. For example, as shown in FIG. 4, human sleep gradually proceeds to a deep sleep stage after falling sleep. Subsequently, human sleep repeatedly alternates between a light sleep stage and the deep sleep stage.

As is already well known, human sleep may be divided into REM sleep and non-REM sleep, and REM sleep may be divided into a total of four stages.

It is well known that REM sleep makes up about 20% to 25% of the entire sleep, and during REM sleep, a characteristic electroencephalogram (EEG) exists, muscle tension is reduced to the lowest level, and characteristic REM is observed.

Further, for example, it is known that people's brains are active while dreaming during REM sleep, and it is also known that a significant change occurs in heart rate and respiration rate during REM sleep.

Also, non-REM sleep may be divided into a first stage, a second stage, a third stage, and a fourth stage, as described above. Sleep in the third stage and the fourth stage is typically referred to as deep sleep, and sleep in the first stage and the second stage is typically referred to as light sleep. Here, it is known that the deep sleep makes up about 15 to 20% of the entire sleep.

During non-REM sleep, muscle tension is reduced and human movement is also reduced. It is also known that a human brain's activity is significantly reduced during non-REM sleep.

In particular, it is known that a heart rate and a respiration rate during non-REM sleep are decreased further than during waking and are relatively regular.

As described above, human sleep exhibits a characteristic heart rate, respiration rate, and movement in each of the stages. Thus, the user terminal 200 or the sleep management server SV may determine a sleep stage of the user U on the basis of the sleep data SD indicating the user U's heart rate, respiration rate, and movement during sleep.

For example, the user terminal 200 or the sleep management server SV may determine whether the sleep of the user U enters or exits a REM sleep stage, and may determine and/or store a time at which the REM sleep stage is entered and a time at which the REM sleep stage is exited.

As another example, the user terminal 200 or the sleep management server SV may determine whether the sleep of the user U enters or exits deep sleep stages (the third and fourth stages) of non-REM sleep and may determine and/or store a time at which the deep sleep stage is entered and a time at which the deep sleep stage is exited.

Also, it is known that a sleeping person consciously or unconsciously wakes up. For example, as shown in FIG. 4, a person enters a waking stage during REM sleep. Also, in the waking stage, a person may consciously or unconsciously wake up, and, while waking, his or her heart beat and respiration rate increase, and his or her movement becomes active.

Accordingly, the user terminal 200 or the sleep management server SV may determine whether the user U wakes from sleep on the basis of the sleep data SD indicating the user U's heart rate, respiration rate, and movement during sleep.

For example, the user terminal 200 or the sleep management server SV may determine whether the user U wakes from the sleep, and may determine and/or store a time at which the user U wakes up. Also, when the user U falls asleep again within a reference time (e.g., 30 minutes) after waking up, the user terminal 200 or the sleep management server SV may determine that the user U continues to sleep, and may determine and/or store a time at which the user U falls asleep again.

Also, when sleep is completed, the user U may wake up and then get out of the bed B. When the user U wakes up and gets out of the bed B, the user terminal 200 or the sleep management server SV may determine whether the user U wakes up and whether the user U gets out of the bed B on the basis of the sleep data SD received from the sleep data acquisition apparatus 100. Also, the user terminal 200 or the sleep management server SV may determine and/or store a time at which the user U wakes up and a time at which the user U gets out of the bed B.

As described above, the user terminal 200 or the sleep management server SV may obtain the sleep information SI of the user U on the basis of the sleep data SD. In detail, the user terminal 200 or the sleep management server SV may obtain sleep time information such as a time at which the user U lies on the bed B, a time at which the user U falls asleep, a time at which the sleep stage of the user U changes, a time at which the user U wakes up for a moment while sleeping, a time at which the user U falls asleep again, a time at which the user U wakes up, and a time at which the user U gets out of the bed B.

Also, the user terminal 200 or the sleep management server SV may generate sleep summary information of the user U on the basis of the sleep time information of the user U. For example, the user terminal 200 or the sleep management server SV may obtain sleep summary information such as a total sleep time, a time it takes the user to fall asleep, the number of times the user wakes up while sleeping, sleep efficiency, a deep sleep time, and a REM sleep time on the basis of the sleep time information of the user U.

Also, the user terminal 200 may display the sleep summary information to the user according to input of the user.

Configurations of the sleep data acquisition apparatus 100 and the user terminal 200 included in the sleep management system 1 will be described below.

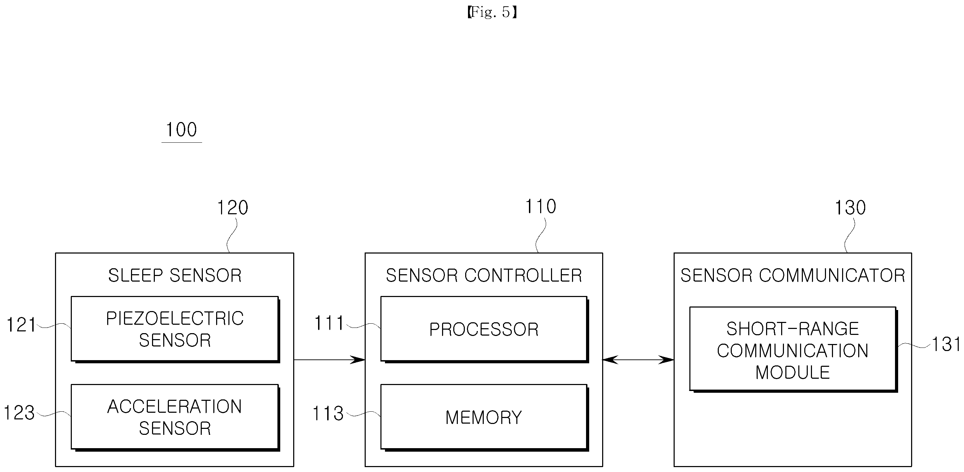

FIG. 5 shows a configuration of a sleep data acquisition apparatus included in a sleep management system according to an embodiment.

As shown in FIG. 5, the sleep data acquisition apparatus 100 may include a sleep sensor 120, a sensor communicator 130, and a sensor controller 110.

The sleep sensor 120 may detect a heart rate, respiration rate, and movement of a user who is lying on a bed and output an electrical signal corresponding to the detected heart rate, respiration rate, and movement of the user to the sensor controller 110.

For example, the sleep sensor 120 may include a piezoelectric sensor 121 configured to detect pressure caused by the heart rate, respiration rate, and movement of the user and output an electrical signal corresponding to the detected pressure. The piezoelectric sensor 121 uses a piezoelectric effect in which electric polarization occurs on a surface of a crystal when a force is applied to the crystal. The piezoelectric sensor 121 generates an alternating current (AC) voltage when pressure is applied thereto, and generates a vibration when the AC voltage is applied thereto.

In detail, when pressure is applied to the piezoelectric sensor 121 by the heart rate, respiration rate, and movement of the user, the piezoelectric sensor 121 may output an electrical signal corresponding to the pressure applied thereto.

As another example, the sleep sensor 120 may include an acceleration sensor 123 configured to detect a vibration caused by the heart rate, respiration rate, and movement of the user and output an electrical signal corresponding to the detected vibration.

The acceleration sensor 123 is a sensor configured to measure a change in speed per unit time. When the acceleration sensor 123 is moved or vibrated by the heart rate, respiration rate, and movement of the user, the acceleration sensor 123 may output an electrical signal corresponding to the movement or vibration.

However, the sleep sensor 120 does not have to include both of the piezoelectric sensor 121 and the acceleration sensor 123. Depending on a designer's selection, the sleep sensor 120 may include either the piezoelectric sensor 121 or the acceleration sensor 123.

The sensor communicator 130 may include a short-range communication module 131 configured to transmit and/or receive data to and/or from the user terminal 200.

The short-range communication module 131 may communicate with a communication partner through various communication schemes. For example, the short-range communication module 131 may communicate with a communication partner through a WiFi communication scheme (IEEE 802.11), a Bluetooth communication scheme (IEEE 802.15.1), a Zigbee communication scheme (IEEE 802.15.4), or the like

However, the short-range communication module 131 does not employ all of the WiFi communication scheme, the Bluetooth communication scheme, and the Zigbee communication scheme and may employ at least one of the WiFi communication scheme, the Bluetooth communication scheme, and the Zigbee communication scheme.

The sensor controller 110 may include a memory 113 configured to store and/or recall programs and data, and a processor 111 configured to process the data according to the programs stored in the memory 113 and control the sleep sensor 120 and the sensor communicator 130.

The memory 113 may store a control program and control data for controlling operation of the sleep data acquisition apparatus 100, or may recall sleep data SD obtained through the sleep sensor 120.

The memory 113 may include a volatile memory such as a static random access memory (S-RAM) and a dynamic RAM (D-RAM) and a non-volatile memory such as a read-only memory (ROM), an erasable programmable ROM (EPROM), an electrically erasable programmable ROM (EEPROM), and a flash memory.

The volatile memory, which is a memory that loses data recalled therein when power is cut off, may temporarily recall programs and data. For example, the volatile memory may recall a control program and control data or recall the sleep data SD obtained by the sleep sensor 120.

The non-volatile memory, which is a memory capable of maintaining data stored therein even though power is cut off, may semi-permanently store programs and data. For example, the non-volatile memory may store a control program and control data for controlling operation of the sleep data acquisition apparatus 100.

The processor 111 may process the sleep data SD input from the sleep sensor 120 according to the control program and control data stored in the memory 113 and transmit the sleep data to the user terminal 200 through the sensor communicator 130.

For example, the processor 111 may obtain an electrical signal output by the sleep sensor 120 every predetermined time interval and generate the sleep data SD by digitalizing the obtained electrical signal. Also, the processor 111 may output the sleep data SD and a control signal to the sensor communicator 130 so that the sensor communicator 130 can transmit the sleep data SD to the user terminal 200.

As described above, the sensor controller 110 may control operation of the sleep sensor 120 and the sensor communicator 130 included in the sleep data acquisition apparatus 100. An operation of the sleep data acquisition apparatus 100, which will be described below, may be interpreted as being controlled by the sensor controller 110.

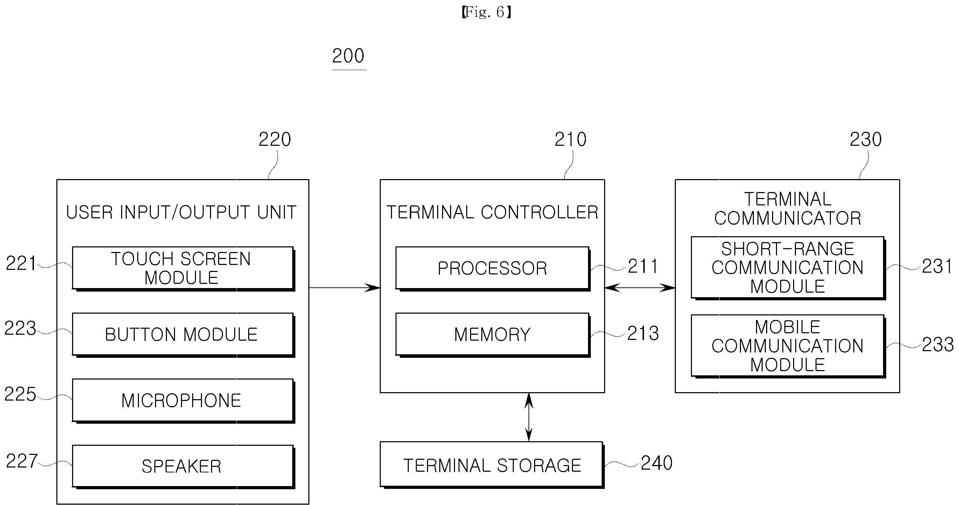

FIG. 6 shows a configuration of a user terminal included in a sleep management system according to an embodiment.

As shown in FIG. 6, the user terminal 200 may include a user input/output unit 220, a terminal communicator 230, a terminal storage 240, and a terminal controller 210.

The user input/output unit 220 may receive a user input from a user and output information corresponding to the user input.

For example, the user input/output unit 220 may include a touch screen module 221 configured to receive a touch input from a user and display information corresponding to the received touch input, a button module 223 configured to receive a predetermined user input from the user, a microphone 225 configured to receive a voice input from the user, and a speaker 227 configured to output sounds.

The touch screen module 221 may receive a touch input from a user and display image information corresponding to the received touch input.

In detail, the touch screen module 221 may receive at least one touch or a continuous touch movement through the user's body (e.g., his or her finger) or a touchable input means (e.g., a stylus pen).

The touch screen module 221 may convert the touch input of the user into a digital signal corresponding to the touch input (e.g., an x coordinate and a y coordinate) and output the digital signal to the terminal controller 210.

The touch screen module 221 may be implemented as a resistive-type module, a capacitive-type module, an infrared-type module, or an acoustic-wave-type module.

The button module 223 may be provided on a front surface, a side surface, or a rear surface of a housing of the user terminal 200, and may include a plurality of buttons capable of receiving a predetermined user input. The button module 223 may include at least one of a power/lock button, a volume button, a menu button, a home button, and a return button.

The terminal communicator 230 may transmit and/or receive data to and/or from the sleep data acquisition apparatus 100 and the sleep management server SV, which have been described above. In addition, the terminal communicator 230 may transmit and/or receive data to and/or from other user terminals.

The terminal communicator 230 may include a short-range communication module 231 configured to transmit and/or receive data to and/or from a communication partner at a location that is relatively near the communication partner, and a mobile communication module 233 configured to transmit and/or receive data to and/or from a communication partner regardless of a distance from the communication partner.

The short-range communication module 231 may communicate with a communication partner through various communication schemes. For example, the short-range communication module 231 may communicate with a communication partner through a WiFi communication scheme (IEEE 802.11), a Bluetooth communication scheme (IEEE 802.15.1), a Zigbee communication scheme (IEEE 802.15.4), or the like

However, the short-range communication module 231 does not employ all of the WiFi communication scheme, the Bluetooth communication scheme, and the Zigbee communication scheme and may employ at least one of the WiFi communication scheme, the Bluetooth communication scheme, and the Zigbee communication scheme.

The mobile communication module 233 may communicate with a communication partner through various communication schemes. For example, the mobile communication module 233 may communicate with the communication partner by using a second generation communication scheme such as time division multiple access (TDMA) and code division multiple access (CDMA), a third generation communication scheme such as wideband code division multiple access (WCDMA), code division multiple access 2000 (CDMA2000), wireless broadband (Wibro), and world interoperability for microwave access (WiMAX), a fourth generation communication scheme such as long term evolution (LTE) and wireless broadband evolution, or the like.

Various application programs and data for performing various functions may be stored in the terminal storage 240 according to a user input received through the user input/output unit 220. For example, an operating system (OS) program for managing configurations and resources (software and hardware) included in the user terminal 200, a video playback program for playing a video and displaying a picture, a word processor program for creating and editing a document, a browser for accessing a WAN such as the Internet, and the like may be stored in the terminal storage 240.

The terminal storage 240 may include a non-volatile memory in which no program or data is lost even when power is cut off. For example, the terminal storage 240 may include a flash memory for easily writing and erasing data or the like.

The terminal controller 210 may include a memory 213 configured to store and/or recall programs and data, and a processor 211 configured to process data according to the program stored in the memory 213 and control the user input/output unit 220, the terminal communicator 230, and the terminal storage 240.

The memory 213 may store a control program and control data for controlling operation of the user terminal 200, or may recall a user input received through the user input/output unit 220, communication data received through the terminal communicator 230, an application program loaded from the terminal storage 240, and the like.

The memory 213 may include a volatile memory such as an S-RAM and a D-RAM and a non-volatile memory such as a ROM, an EPROM, an EEPROM, and a flash memory.

The volatile memory, which is a memory that loses data recalled therein when power is cut off, may temporarily recall programs and data. The volatile memory may store an application program or may recall the sleep data SD received from the sleep data acquisition apparatus 100.

The non-volatile memory, which is a memory that maintains data stored therein even though power is cut off, may semi-permanently store programs and data. For example, the non-volatile memory may store a booting program and data for booting the user terminal 200 and the sleep data SD received from the sleep data acquisition apparatus 100.

The processor 211 may process data according to a program recalled in the memory 213 and control the user input/output unit 220, the terminal communicator 230, and the terminal storage 240 according to the processed data.

For example, the processor 211 may process the sleep data SD received through the terminal communicator 230 to generate sleep time information of the user. Also, the processor 211 may process the sleep time information of the user to generate sleep summary information of the user. Also, the processor 211 may output the sleep summary information of the user and a control signal to the user input/output unit 220 so that the sleep summary information can be displayed through the touch screen module 221.

As another example, the processor 211 may output the sleep data SD and a control signal to the terminal communicator 230 so that the sleep data SD can be transmitted to the sleep management server SV. Also, when the sleep information SI is received from the sleep management server SV, the processor 211 may extract the sleep summary information from the sleep information SI. Also, the processor 211 may output the sleep summary information and a control signal to the user input/output unit 220 so that the sleep summary information can be displayed through the touch screen module 221.

As described above, the terminal controller 210 may control operation of the user input/output unit 220, the terminal communicator 230, and the terminal storage 240 included in the user terminal 200.

An operation of the user terminal 200, which will be described below, may be interpreted as being controlled by the terminal controller 210.

In particular, the control operation of the terminal controller 210 may be implemented as a sleep management application program. The user may download an application program through a WAN or a local area network (LAN). When the user executes the sleep management application program, the terminal controller 210 may execute a control operation that will be described below.

Configurations and operation of the sleep data acquisition apparatus 100 and the user terminal 200 included in the sleep management system 1 have been described above.

A method of providing sleep information of the sleep management system 1 will be described below.

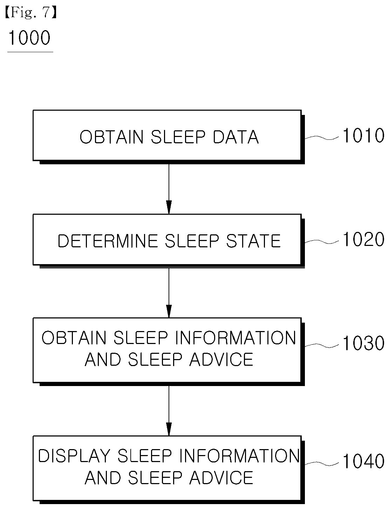

FIG. 7 shows an example of a sleep information display operation of a sleep management system according to an embodiment.

A sleep information display operation 1000 of the sleep management system 1 will be described with reference to FIG. 7.

The sleep management system 1 obtains sleep data SD of a user (1010).

The sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep data acquisition apparatus 100 may transmit the sleep data SD to the user terminal 200 whenever the user's heart rate, respiration rate, and movement are detected or whenever a request for the sleep data SD is received from the user terminal 200 in addition to every predetermined time interval.

The sleep management system 1 determines a sleep state SS of the user (1020).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

For example, the user terminal 200 may extract the user's heart rate, respiration rate, and movement from the sleep data SD and determine whether the user lies on a bed, whether the user falls asleep, in which sleep stage the user is, whether the user wakes up briefly while sleeping, whether the user falls asleep again, whether the user completely wakes up, whether the user gets out of bed, and the like, on the basis of the extracted heart rate, respiration rate, and movement.

As another example, the user terminal 200 may transmit the sleep data SD to the sleep management server SV and receive a sleep state SS, such as whether the user lies on a bed, whether the user falls asleep, in which sleep stage the user is, whether the user wakes up briefly while sleeping, whether the user falls asleep again, whether the user completely wakes up, and whether the user gets out of bed, from the sleep management server SV.

Also, the sleep management server SV may determine the sleep state SS of the user on the basis of the sleep data SD received from the user terminal 200.

For example, the sleep management server SV may extract the user's heart rate, respiration rate, and movement from the sleep data SD and determine whether the user lies on a bed, whether the user falls asleep, in which sleep stage the user is, whether the user wakes up briefly while sleeping, whether the user falls asleep again, whether the user completely wakes up, whether the user gets out of bed, and the like on the basis of the extracted heart rate, respiration rate, and movement.

The sleep management system 1 obtains sleep information SI of the user and sleep advice (1030).

Here, the sleep information SI may include sleep summary information that summarizes sleep quality of the user and information that may be additionally obtained from the sleep summary information.

The user terminal 200 of the sleep management system 1 may directly determine the sleep state SS of the user on the basis of the sleep data SD of the user, or may receive the sleep information SI of the user from the sleep management server SV.

For example, the user terminal 200 may accumulate the sleep state SS of the user. Subsequently, the user terminal 200 may obtain sleep time information such as a time at which the user lies on a bed, a time at which the user falls asleep, a time at which the sleep stage of the user changes, a time at which the user wakes up briefly while sleeping, a time at which the user falls asleep again, a time at which the user wakes up, and a time at which the user gets out of bed on the basis of the accumulated sleep state SS of the user.

Also, the user terminal 200 may obtain sleep summary information such as a total sleep time of the user, a time it takes the user to fall asleep, the number of times the user wakes up while sleeping, sleep efficiency, a deep sleep time, a REM sleep time, or the like on the basis of the sleep time information.

As another example, the user terminal 200 may receive sleep summary information such as a total sleep time, a time it takes the user to fall asleep, the number of times the user wakes up while sleeping, sleep efficiency, a deep sleep time, a REM sleep time, or the like of the user from the sleep management server SV.

Also, the sleep management server SV may generate the sleep information SI of the user on the basis of the sleep data SD received from the user terminal 200.

For example, the sleep management server SV may accumulate the sleep state SS of the user and generate sleep time information of the user on the basis of the accumulated sleep state SS of the user.

Also, the sleep management server SV may generate the sleep summary information of the user on the basis of the sleep time information.

The sleep advice may include common sense about sleep and advice of a sleep specialist or a metabolic specialist, and may change depending on the type of sleep of the user. For example, the type of sleep of the user is divided into a plurality of sleep type groups, and appropriate sleep advice may be provided for each of the sleep type groups.

The user terminal 200 of the sleep management system 1 may determine the type of sleep of the user on the basis of the sleep information SI, and may obtain appropriate sleep advice for the type of sleep. The sleep advice may be prestored in the storage 240 of the user terminal 200, or may be received from the sleep management server SV.

Also, the sleep management server SV of the sleep management system 1 may also determine the type of sleep of the user on the basis of the sleep information SI and generate appropriate sleep advice for the type of sleep.

The sleep management system 1 displays the sleep information SI of the user and the sleep advice (1040).

The user terminal 200 of the sleep management system 1 may display the sleep information SI of the user in various forms depending on a user input.

For example, the user terminal 200 may display the sleep summary information as it is, may display the sleep summary information by using a change in sleep state over time, or may display the sleep summary information on a date basis. In this case, information indicating the change in sleep state over time is referred to as daily sleep information, and information indicating monthly sleep summary is referred to as monthly sleep information.

In other words, the user terminal 200 may selectively display the sleep summary information, the daily sleep information, the monthly sleep information, and the sleep advice according to a user input.

Also, the user terminal 200 may display the sleep summary information, the daily sleep information, the monthly sleep information, and the sleep advice of the user through a graphic user interface.

The graphic user interface displayed by the user terminal 200 in association with the sleep information SI and the sleep advice will be described in detail below.

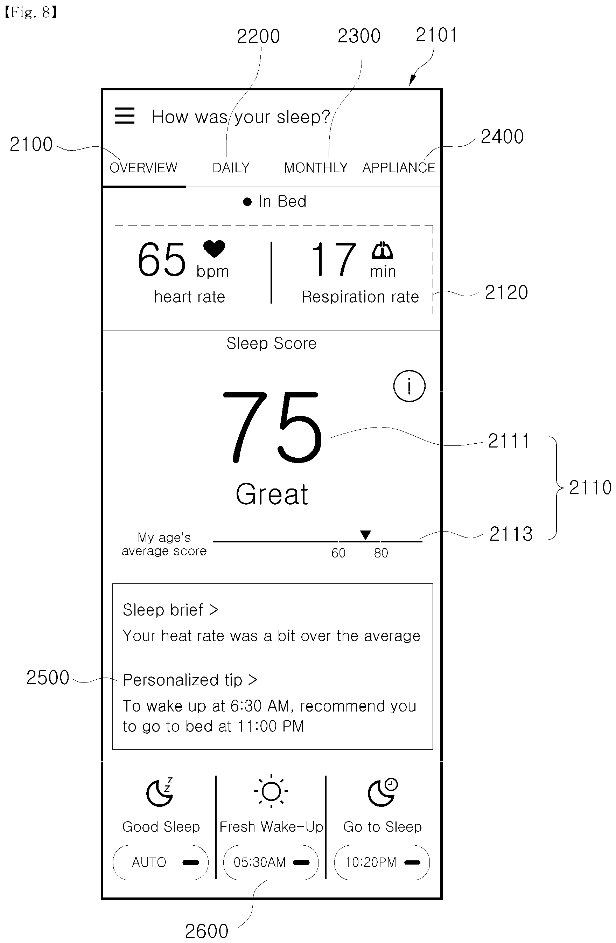

FIG. 8 shows an example of a summary sleep information screen displayed by a sleep management system according to an embodiment.

A summary sleep information screen 2101 shown in FIG. 8 may be displayed on the user terminal 200 according to a user input.

A plurality of menus 2100, 2200, 2300, and 2400 for selecting information displayed on the user terminal 200 may be displayed at one side of the summary sleep information screen 2101.

In detail, the plurality of menus 2100, 2200, 2300, and 2400 include a sleep summary menu 2100 for displaying sleep summary information, a daily sleep summary menu 2200 for displaying sleep summary information associated with the current date, a monthly sleep summary menu 2300 for displaying sleep summary information associated with a month of the current date, and an electronic device menu 2400 for setting operation of an electronic device.

When the user touches one of the menus 2100, 2200, 2300, and 2400, a screen corresponding to the touched menu may be displayed.

Also, sleep data 2120 may be displayed at the one side of the summary sleep information screen 2101.

The sleep data 2120 may include a heart rate per minute, a respiration rate per minute, and the like during sleep.

Also, a sleep score 2110 may be displayed at the one side of the summary sleep information screen 2101.

The sleep score 2110 represents sleep quality of the user in a numerical form on the basis of the sleep data 2120. In detail, in order to calculate the sleep score 2110, the user terminal 200 generates sleep summary information including a total sleep time, a time it takes the user to fall asleep, the number of times the user wakes up while sleeping, sleep efficiency, a deep sleep time, and a REM sleep time of the user on the basis of the sleep data 2120. Subsequently, the user terminal 200 may represent items included in the sleep summary information in numerical form as numbers, weight the numbers, and then sum the weighted numbers.

The user may quantitatively confirm his or her sleep quality through the sleep score 2110. Also, the user may quantitatively compare sleep of the current day and sleep of the previous day through the sleep score 2110.

The sleep score 2110 may be displayed in various ways.

For example, the sleep score 2110 may be simply displayed as a numeric type sleep score 2111 or may be displayed as a graphic type sleep score 2113.

When the sleep score 2110 is displayed as the graphic type sleep score 2113, an average sleep score of an age group in which the user is included may also be displayed. By the average sleep score of the age group in which the user is included being displayed, the user may determine to what level his or her sleep quality corresponds.

Also, a warning message or a congratulatory message may be displayed on the summary sleep information screen 2101 according to the sleep score 2110. For example, when the sleep score 2110 of the current date is significantly decreased relative to the sleep score 2110 of the previous date, a warning message for alerting the user may be displayed in the summary sleep information screen 2101. Also, when the sleep score 2110 of the current date is significantly increased relative to the sleep score 2110 of the previous date, a congratulatory message may be displayed in the sleep information display screen 2101.

Also, an advice menu 2500 for displaying sleep advice and an alarm time setting menu 2600 for setting a wake-up time may be displayed at one side of the summary sleep information screen 2101.

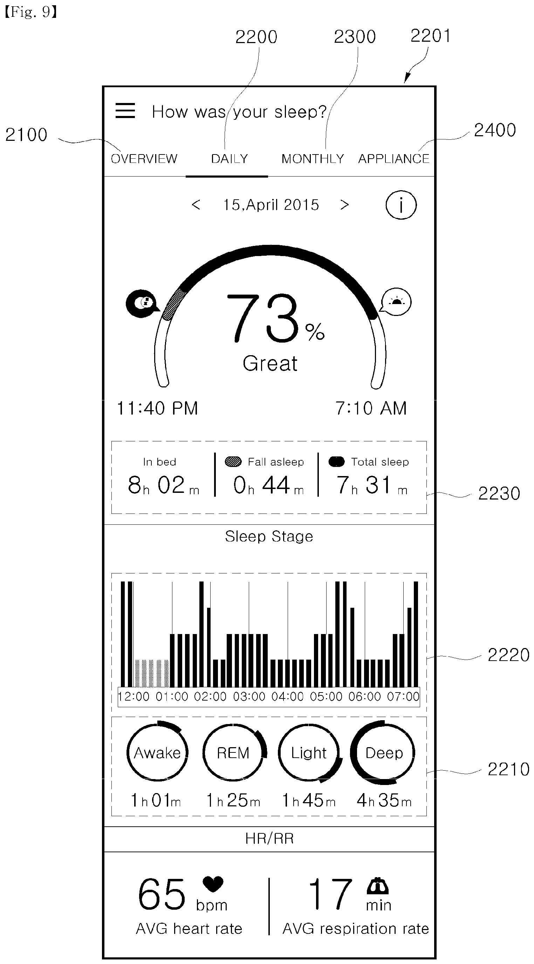

FIG. 9 shows an example of a daily sleep information screen displayed by a sleep management system according to an embodiment.

A daily sleep information screen 2201 shown in FIG. 9 may be displayed on the user terminal 200 according to a user input. In detail, when a user touches the daily sleep summary menu 2200, the user terminal 200 may display the daily sleep information screen 2201.

A plurality of menus 2100, 2200, 2300, and 2400 for selecting displayed information may be displayed at one side of the daily sleep information screen 2201.

First daily sleep information 2210 may be displayed at the one side of the daily sleep information screen 2201. The first daily sleep information 2210 may include an awake time during sleep, a REM sleep time, a light sleep time, a deep sleep time, and the like.

Also, second daily sleep information 2220 may be displayed at the one side of the daily sleep information screen 2201. The second daily sleep information 2220 may include a sleep state of the user over time. For example, the second daily sleep information 2220 may indicate whether the user is awake, whether the user is in REM sleep, whether the user is in light sleep, or whether the user is in deep sleep depending on a sleep time of the user.

Also, third daily sleep information 2230 may be displayed at the one side of the daily sleep information screen 2201. The third daily sleep information 2230 may include a time at which the user is in a bed, a time it takes the user to fall asleep, a total sleep time, and the like.

The user may confirm a pattern and quality of sleep of the current day on the basis of sleep summary information of the current date displayed in the daily sleep information screen 2201.

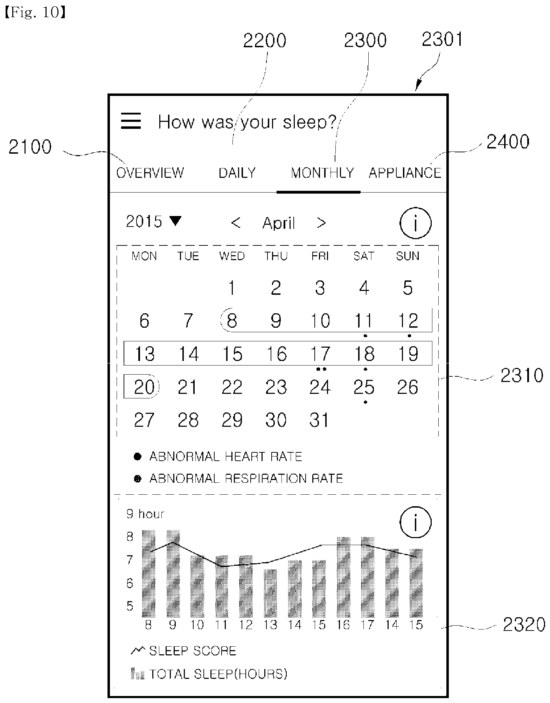

FIG. 10 shows an example of a monthly sleep information screen displayed by a sleep management system according to an embodiment.

A monthly sleep information screen 2301 shown in FIG. 10 may be displayed on the user terminal 200 according to a user input. In detail, when a user touches the monthly sleep summary menu 2300, the user terminal 200 may display the monthly sleep information screen 2301.

The plurality of menus 2100, 2200, 2300, and 2400 for selecting displayed information may be displayed at one side of the monthly sleep information screen 2301.

Also, first monthly sleep information 2310 may be displayed at the one side of the monthly sleep information screen 2301.

The first monthly sleep information 2310 may display sleep information of all dates included in a month selected by the user in the form of a calendar.

For example, the first monthly sleep information 2310 displayed on a calendar may include an indication of whether a sleep score for each of the dates is greater or smaller than an average sleep score.

Also, second monthly sleep information 2320 may be displayed at the one side of the monthly sleep information screen 2301.

The second monthly sleep information 2320 may include a sleep score for each of the dates, and the sleep score for each of the dates may be displayed using a graph.

The user may confirm pattern and quality of sleep of the current day on the basis of monthly sleep summary information displayed in the monthly sleep information screen 2301.

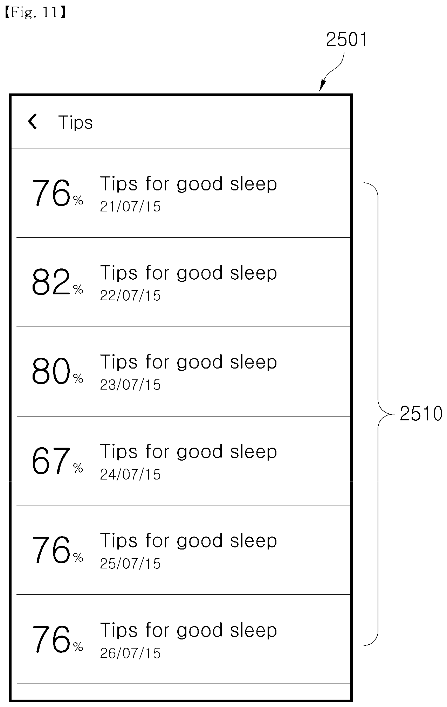

FIG. 11 shows an example of a sleep advice screen displayed by a sleep management system according to an embodiment.

A sleep advice screen 2501 shown in FIG. 11 may be displayed on the user terminal 200 according to a user input. In detail, when a user touches an advice menu 2500, the user terminal 200 may display the sleep advice screen 2501.

A plurality of pieces of sleep advice 2510 may be displayed in the sleep advice screen 2501 on a date basis.

The sleep advice 2510 may be written on the basis of the sleep information SI of the user.

For example, the type of sleep of the user may be classified into a plurality of sleep type groups by using indices such as a total sleep time, a time it takes the user it takes the user to fall asleep, the number of times the user wakes up while sleeping, sleep efficiency, a deep sleep time, a REM sleep time, and the like.

The sleep type groups may be classified on the basis of a plurality of pieces of sleep summary information as well as one piece of sleep summary information. For example, a first group and a second group may be classified according to the total sleep time. In detail, the first group may be a sleep type group in which the total sleep time is 20% longer than an average sleep time, and the second group may be a sleep type group in which the total sleep time is 20% shorter than the average sleep time. Also, the third group may be classified on the basis of the total sleep time and the time it takes the user to fall asleep. In detail, the third group may be a type in which the total sleep time is within ±20% of the average sleep time and the time it takes the user to fall asleep is within one hour.

Also, the plurality of sleep type groups may include sleep-associated advice such as a sleep time, nutrition, a sleep environment, sleep hygiene, activities before sleep, stress, knowledge about sleep, jet lag, insomnia, and sleep disorder appropriate for each of the sleep types.

Also, the user terminal 200 may determine a sleep type group to which the user belongs on the basis of the sleep information SI of the user. Also, the user terminal 200 may display the sleep advice 2510 according to the determined sleep type group.

Also, the sleep advice 2510 may include normal advice and specialist advice.

The normal advice may include sleep advice such as a sleep score, a change of the sleep score, a sleep habit problem, and the like.

Also, the specialist advice 2530 may include sleep advice written by a sleep specialist or a metabolic specialist.

The user may obtain appropriate sleep advice for the type of sleep of the user through sleep advice displayed in the sleep advice screen 2501, and may actively want to improve his or her sleep quality.

Another example of a sleep information display operation of the sleep management system 1 will be described below.

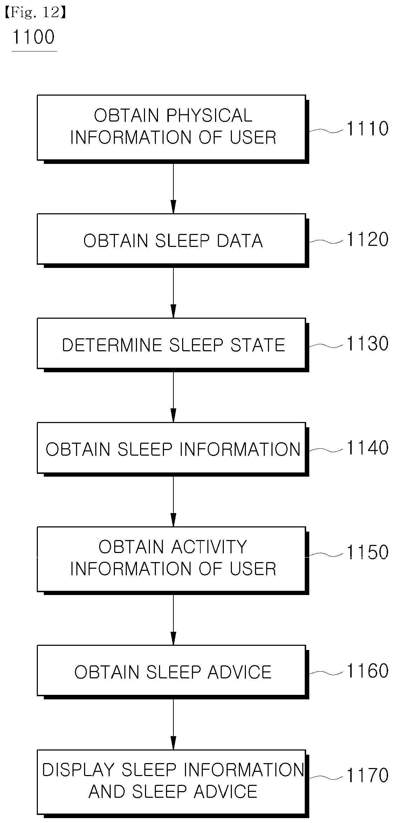

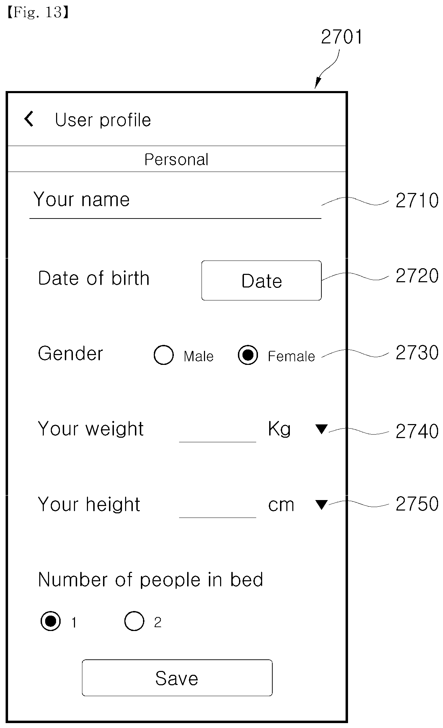

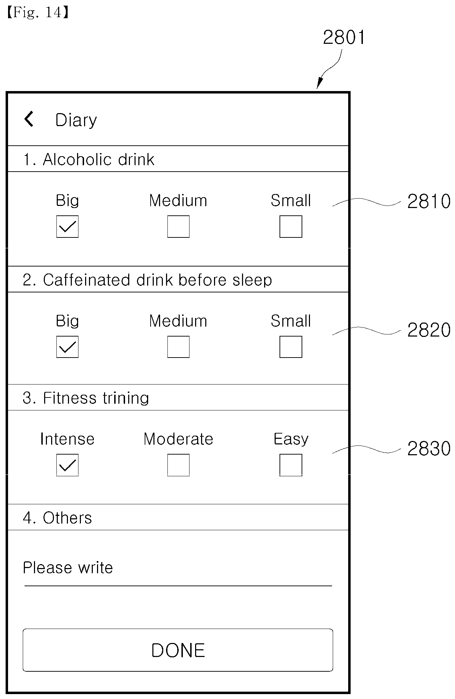

FIG. 12 shows another example of a sleep information display operation of a sleep management system according to an embodiment. Also, FIG. 13 shows an example of a screen in which a sleep management system obtains physical information of a user according to an embodiment, and FIG. 14 shows an example of a screen in which a sleep management system obtains sleep-associated activity information of a user according to an embodiment.

A sleep information display operation 1100 of the sleep management system 1 will be described with reference to FIGS. 12, 13, and 14.

The sleep management system 1 obtains physical information of a user from the user (1110).

In order to obtain accurate sleep information of the user and provide accurate sleep advice to the user, the sleep management system 1 may obtain the physical information of the user from the user through the user terminal 200. Also, the user terminal 200 may transmit the obtained physical information to the sleep management server SV.

Human sleep may have different patterns depending on physical conditions such as gender, height, and weight. Also, necessary sleep advice may change depending on human physical conditions. Accordingly, the sleep management system 1 may obtain information regarding physical conditions of the user to provide more accurate sleep advice to the user.

For example, the user terminal 200 may display a physical information input screen 2701, as shown in FIG. 13.

A name input region 2710 for receiving the user's name, a birthday input region 2720 for receiving the user's birthday, a gender input region 2730 for receiving the user's gender, a weight input region 2740 for receiving the user's weight, and a height input region 2750 for receiving the user's height may be displayed in the physical information input screen 2701.

Physical information input by the user may be used to display the above-described pieces of sleep advice.

The sleep management system 1 obtains sleep data SD of the user (1120).

The sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep management system 1 determines a sleep state SS of the user on the basis of the sleep data SD (1130).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

Also, the sleep management server SV may determine the sleep state SS of the user on the basis of the sleep data SD received from the user terminal 200.

The sleep management system 1 obtains sleep information SI of the user (1140).

Here, the sleep information SI may include sleep summary information that summarizes sleep quality of the user and information that may be additionally obtained from the sleep summary information.

The user terminal 200 of the sleep management system 1 may directly determine the sleep state SS of the user on the basis of the sleep data SD of the user, or may receive the sleep information SI of the user from the sleep management server SV.

Also, the sleep management server SV may generate the sleep information SI of the user on the basis of the sleep data SD received from the user terminal 200.

The sleep management system 1 obtains sleep-associated activity information of the user (1150).

In order to obtain accurate sleep information of the user and provide accurate sleep advice to the user, the sleep management system 1 may obtain sleep-associated activity information of the user from the user through the user terminal 200. Here, the sleep-associated activity information includes activities that may affect the user's sleep. Drinking, eating, exercising, and the like before sleep may be included in the sleep-associated activity information.

For example, the user terminal 200 may display an activity information input screen 2801, as shown in FIG. 14.

A drinking input region 2810 for receiving an indication of whether the user drinks, a stimulant intake input region 2820 for receiving an indication of whether the user takes a stimulant, and an exercise input region 2830 for receiving an indication of whether the user exercises may be displayed in the activity information input screen 2801.

As is well known, human sleep is affected by drinking, intaking a stimulant, exercising, and the like before sleep. In particular, drinking and intaking a stimulant before sleep may have a significant influence on sleep quality. For example, drinking and intaking a stimulant may decrease a ratio of deep sleep time to the entire sleep time.

Accordingly, in order to provide appropriate sleep advice, the sleep management system 1 may receive an indication of whether the user drinks, an indication of whether the user takes a stimulant, and an indication of whether the user exercises through the user terminal 200.

The sleep management system 1 obtains sleep advice for the user (1160).

The sleep advice may include common sense about sleep and advice of a sleep specialist or a metabolic specialist, and may change depending on the type of sleep of the user. For example, the type of sleep of the user is divided into a plurality of sleep type groups, and appropriate sleep advice may be provided for each of the sleep type groups.

Also, the sleep advice may change depending on the activity information of the user. For example, when a user drinks before sleep, advice on the association between drinking and sleep may be provided to the user. Also, when a user takes a stimulant before sleep, advice on the association between taking a stimulant and sleep may be provided to the user.

In detail, the user terminal 200 of the sleep management system 1 may obtain sleep advice on the basis of the sleep information SI of the user and the activity information of the user. The sleep advice may be prestored in the storage 240 of the user terminal 200, or may be received from the sleep management server SV.

Also, the sleep management server SV of the sleep management system 1 may generate sleep advice on the basis of the sleep information SI of the user and the activity information of the user.

The sleep management system 1 displays the sleep information SI of the user and the sleep advice (1170).

The user terminal 200 of the sleep management system 1 may display the sleep information SI of the user in various forms depending on a user input. In other words, the user terminal 200 may selectively display the sleep summary information, the daily sleep information, the monthly sleep information, and the sleep advice according to the user input.

For example, the user terminal 200 may display the summary sleep information screen 2001 (see FIG. 8), the daily sleep information screen 2201 (see FIG. 9), the monthly sleep information screen 2301 (see FIG. 10), or the sleep advice screen 2501 (see FIG. 11) depending on a touch input of the user.

In particular, the sleep advice screen 2501 (see FIG. 11) may provide various pieces of sleep advice to the user on the basis of the sleep information of the user and the activity information of the user.

For example, when a user drinks before sleep, advice on the association between drinking and sleep may be displayed in the sleep advice screen 2501 (see FIG. 11). Also, when a user takes a stimulant before sleep, advice on the association between taking a stimulant and sleep may be displayed in the sleep advice screen 2501 (see FIG. 11).

The user may obtain sleep advice appropriate for the type of sleep of the user through the sleep advice displayed in the sleep advice screen 2501 (see FIG. 11). A user who drinks, takes a stimulant, or exercises before sleep may obtain advice on drinking, taking a stimulant, and exercising before sleep.

Sharing of sleep information through the sleep management system 1 will be described below.

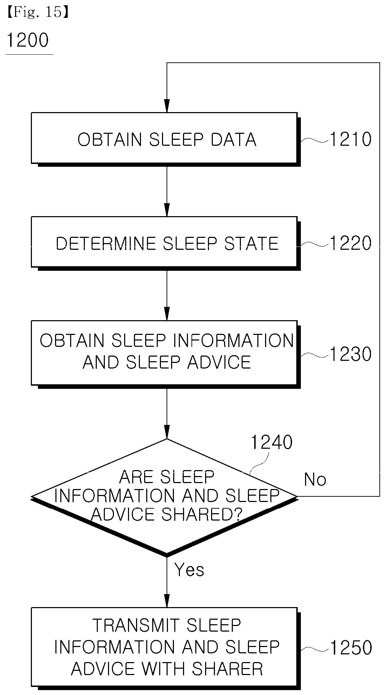

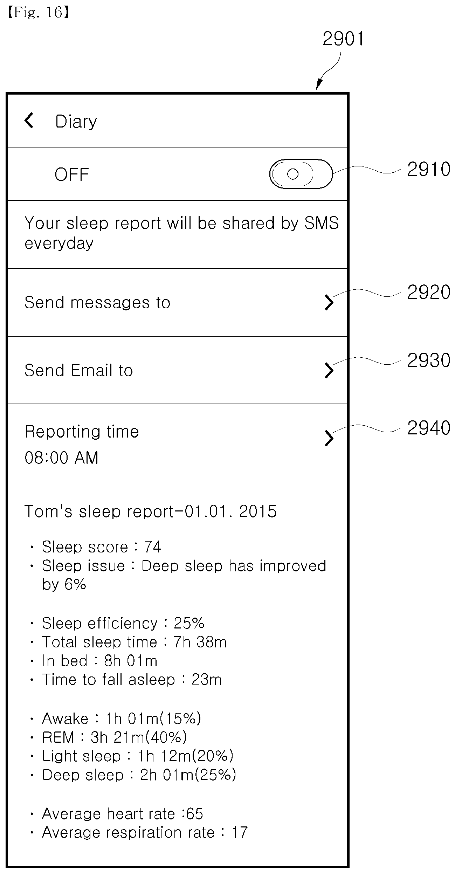

FIG. 15 shows an example of a sleep information sharing operation of a sleep management system according to an embodiment, and FIG. 16 shows an example of a screen in which a sleep management system obtains settings about whether to share sleep information according to an embodiment.

A sleep information sharing operation 1200 of the sleep management system 1 will be described with reference to FIGS. 15 and 16.

The sleep management system 1 obtains sleep data SD of a user (1210).

The sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep management system 1 determines a sleep state SS of the user (1220).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

Also, the sleep management server SV may determine the sleep state SS of the user on the basis of the sleep data SD received from the user terminal 200.

The sleep management system 1 obtains sleep information SI of the user and sleep advice (1230).

Here, the sleep information SI may include sleep summary information that summarizes sleep quality of the user and information that may be additionally obtained from the sleep summary information.

The user terminal 200 of the sleep management system 1 may directly determine the sleep state SS of the user on the basis of the sleep data SD of the user, or may receive the sleep information SI of the user from the sleep management server SV.

Also, the sleep management server SV may generate the sleep information SI of the user on the basis of the sleep data SD received from the user terminal 200.

The sleep advice may include common sense about sleep and advice of a sleep specialist or a metabolic specialist, and may change depending on the type of sleep of the user. For example, the type of sleep of the user is divided into a plurality of sleep type groups, and appropriate sleep advice may be provided for each of the sleep type groups.

The user terminal 200 of the sleep management system 1 may determine the type of sleep of the user on the basis of the sleep information SI, and may obtain appropriate sleep advice for the type of sleep. The sleep advice may be prestored in the storage 240 of the user terminal 200, or may be received from the sleep management server SV.

Also, the sleep management server SV of the sleep management system 1 may also determine the type of sleep of the user on the basis of the sleep information SI and generate appropriate sleep advice for the type of sleep.

The sleep management system 1 determines whether to share the sleep information SI of the user and the sleep advice with other users (1240).

The user terminal 200 of the sleep management system 1 may determine whether to share the sleep information SI of the user and the sleep advice with other users on the basis of a setting value related to the sharing of the sleep information set by the user.

The setting value related to the sharing of the sleep information may include an indication of whether to share the sleep information SI, a person with whom the sleep information SI is to be shared, and a time at which the sleep information SI is to be shared.

In order to receive the setting value for sharing the sleep information, the user terminal 200 may display an information sharing setting screen 2901.

As shown in FIG. 15, the information sharing setting screen 2901 may include an information sharing on/off region 2910 for receiving an indication of whether to share the sleep information SI, a first sharer setting region 2920 and a second sharer setting region 2930 for setting a sharer of the sleep information SI, and a sharing time setting region 2940 for setting a sharing time for the sleep information SI.

The information sharing on/off region 2910 may be provided in the form of a slide switch, and the sharing of sleep information may be activated or deactivated depending on a touch input of the user.

Different sharers may be input to the first and second sharer setting regions 2920 and 2930 depending on a sharing method for the sleep information SI. For example, a first sharer with which the sleep information is to be shared may be input to the first sharer setting region 2920 through a short message service, and a second sharer with which the sleep information is to be shared may be input to the second sharer setting region 2930 through an e-mail.

Also, identification information of a sharer, identification information of a user terminal of the sharer, and the like may be input to the first and second sharer setting regions 2920 and 2930. For example, an ID of a sleep management application of the sharer, an e-mail address of the sharer, a user terminal number of the sharer, a sharer ID of a social network service (SNS), or the name of the sharer may be input to the sharer setting region 2920.

A time at which the sleep information SI of the user is to be transmitted to the sharer may be input to the sharing time setting region 2940.

In this case, shareable information may include sleep summary information, daily sleep information, monthly sleep information, and sleep advice. However, the user may set information to be shared or may set information not to be shared.

For example, when the user sets sleep summary information as the sleep information to be shared, only the sleep information is shared with the sharer. Also, when the user sets sleep advice as the information not to be shared, the sleep advice is not shared with the sharer.

When the sleep information SI of the user and the sleep advice are not shared with other users (no in 1240), the user terminal 200 repeatedly obtains the sleep data, determines the sleep state, and obtains the sleep information.

When the sleep information SI of the user and the sleep advice are shared with other users (yes in 1240), the sleep management system 1 transmits the sleep information SI of the user and the sleep advice to the sharer (1250).

In detail, when the user allows the sharing of the sleep information SI and the current time reaches the sharing time, the user terminal 200 may transmit the sleep information SI of the user and the sleep advice to the sharer according to the identification information of the sharer or the identification information of the user terminal of the sharer that is input by the user.

For example, when the ID of the sleep management application of the sharer is input, the user terminal 200 may transmit the sleep information SI of the user and the sleep advice through the sleep management server SV by using an account of the sleep management application of the sharer.

Also, when the e-mail address of the sharer is input, the user terminal 200 may transmit the sleep information SI of the user and the sleep advice by using the input e-mail address of the sharer.

Also, when a user terminal number of the sharer is input, the user terminal 200 may transmit the sleep information SI of the user and the sleep advice through a text message by using the input number.

Also, when a sharer ID of a social network service is input, the user terminal 200 may post the sleep information SI of the user and the sleep advice by using a sharer account of a social network service application.

When the name of the sharer is input, the user terminal 200 may search a contact list to obtain the e-mail address of the sharer or the user terminal number of the sharer. Also, the user terminal 200 may transmit the sleep information SI of the user and the sleep advice by using the e-mail address of the sharer, or may transmit the sleep information SI of the user and the sleep advice to the user terminal of the sharer through a text message.

Like this, the user terminal may transmit the sleep information SI of the user and the sleep advice to the sharer through various methods depending on a format of identification information input to the first and second sharer setting regions 2920 and 2930.

An example of the sleep information SI of the user and the sleep advice being shared by the user terminal 200 transmitting the sleep information SI of the user and the sleep advice to the sharer has been described above, but the present disclosure is not limited thereto.

For example, the sleep management server SV may directly transmit the sleep information SI of the user and the sleep advice to the sharer.

The sleep management server SV may obtain the sleep data SD from the user terminal 200 or the sleep data acquisition apparatus 100 and determine the sleep state SS of the user on the basis of the sleep data SD or generate the sleep information SI of the user and the sleep advice.

Also, the sleep management server SV may transmit the sleep information SI of the user and the sleep advice to the sharer according to identification information of the sharer that is input by the user through the information sharing setting screen 2901 of the user terminal.

For example, when the ID of the sleep management application of the sharer is input, the sleep management server SV may transmit the sleep information SI of the user and the sleep advice by using the account of the application management application of the sharer.

Also, when the e-mail address of the sharer is input, the sleep management server SV may transmit the sleep information SI of the user and the sleep advice by using the input e-mail address of the sharer.

Also, when the user terminal number of the sharer is input, the sleep management server SV may transmit the sleep information SI of the user and the sleep advice through a text message by using the input number.

Also, when a sharer ID of a social network service is input, the sleep management server SV may post the sleep information SI of the user and the sleep advice by using a sharer account of a social network service application.

As described above, the user terminal 200 and the sleep management server SV of the sleep management system 1 may share the sleep information of the user with the sharer.

Also, the user may receive sleep information from a third party in addition to sharing his or her sleep information with the third party.

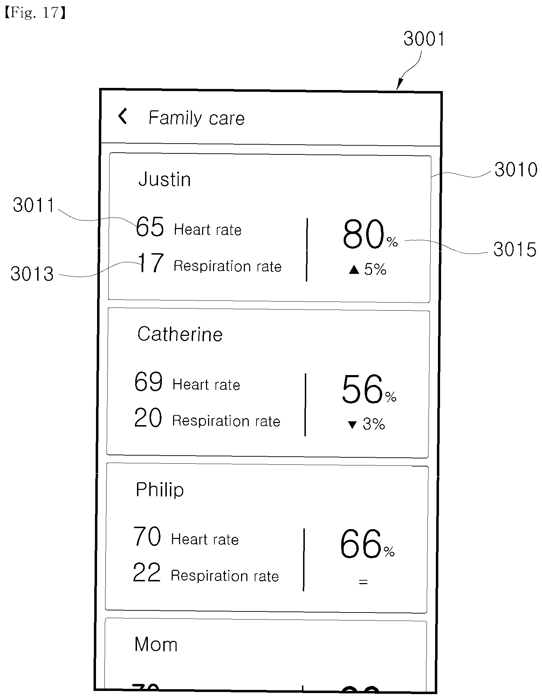

FIG. 17 shows an example in which a sleep management system displays sleep information shared with a third party according to an embodiment.

The user terminal 200 may display a third-party sleep management screen 3001, as shown in FIG. 17.

Third-party sleep information 3010 received from a plurality of third parties may be displayed in the third-party sleep management screen 3001.

The third-party sleep information 3010 may include a heart rate per minute 3011, a respiration rate per minute 3013, and a sleep score 3015 of the third party.

The user may confirm sleep information of the third party and determine sleep quality of the third party, a sleep disorder of the third party, or the like through the third-party sleep management screen 3001.

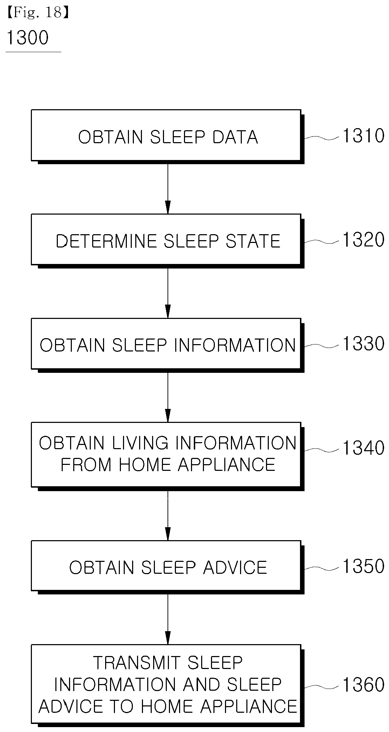

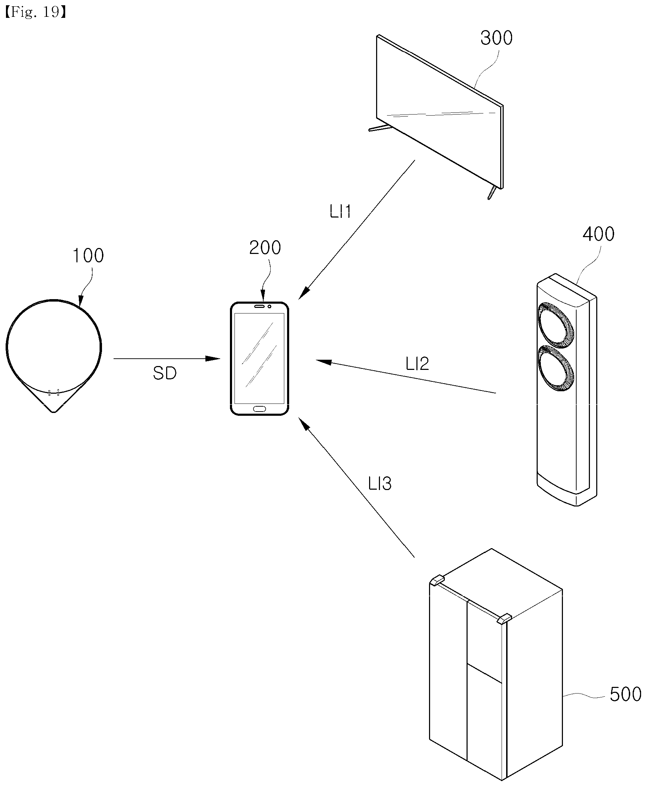

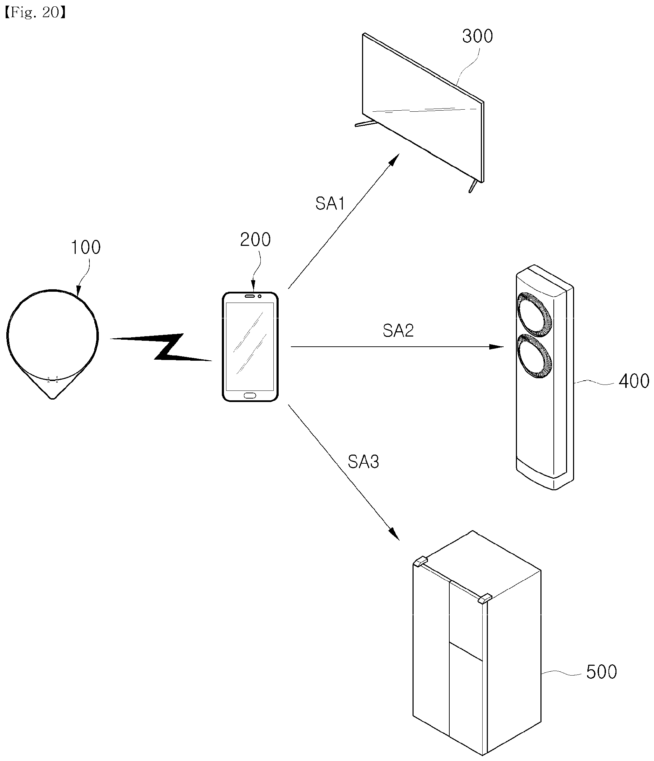

FIG. 18 shows an example of an interoperation with an electronic device of a sleep management system according to an embodiment. Also, FIG. 19 shows an example in which a sleep management system collects data from an electronic device according to an embodiment, and FIG. 20 shows an example in which a sleep management system provides sleep information to an electronic device according to an embodiment.

An interoperation 1300 of the sleep management system 1 with electronic devices 300, 400, and 500 will be described with reference to FIGS. 18, 19, and 20.

The sleep management system 1 obtains sleep data SD of a user (1310).

The sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep management system 1 determines a sleep state SS of the user (1320).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

Also, the sleep management server SV may determine the sleep state SS of the user on the basis of the sleep data SD received from the user terminal 200.

The sleep management system 1 obtains sleep information of the user (1330).

Here, the sleep information SI may include sleep summary information that summarizes sleep quality of the user and information that may be additionally obtained from the sleep summary information.

The user terminal 200 of the sleep management system 1 may directly determine the sleep state SS of the user on the basis of the sleep data SD of the user, or may receive the sleep information SI of the user from the sleep management server SV.

Also, the sleep management server SV may generate the sleep information SI of the user on the basis of the sleep data SD received from the user terminal 200.

The sleep management system 1 obtains living information LI1, LI2, and LI3 of the user from the electronic devices 300, 400, and 500 (1340).

Here the electronic devices 300, 400, and 500 may each refer to any electrical device the user may access.

For example, the electronic devices 300, 400, and 500 may include a home medical instrument, an electric stove, an electric burner, a washing machine, a vacuum cleaner, an air purifier, an audio output device, a user terminal, and a vehicle as well as an image display apparatus 300, an air conditioner 400, and a refrigerator 500 shown in FIG. 19.

Also, the living information LI1, LI2, and LI3 indicates user inputs that are input to the electronic devices 300, 400, and 500 by the user or user-associated data detected by the electronic devices 300, 400, and 500.

For example, the living information LI1, LI2, and LI3 may include a blood glucose level, blood pressure, a body temperature, electrocardiogram, a glycosylated hemoglobin (HbA1c) level, a heart rate, an oxygen saturation level, a stress index, and the like, which may be obtained by a home medical device or the like.

Also, the living information LI1, LI2, and LI3 may include food intake, alcohol intake, caffeine intake, nutrition information of ingested food, calories of ingested food, and the like, which may be obtained by a refrigerator, an electric stove, an electric burner, and the like.

Also, the living information LI1, LI2, and LI3 may include the number of steps, an exercise time, the type of exercise (aerobic exercise, anaerobic exercise, and the like), burned calories, and the like, which may be obtained by a wearable device or the like that may be worn by the user.

In addition, the living information LI1, LI2, and LI3 may include a location of the user, a schedule of the user, an ultraviolet index, weather, temperature, and the like, which may be obtained by the user terminal 200.

As shown in FIG. 19, the plurality of electronic devices 300, 400, and 500 may transmit the living information LI1, LI2, LI3 of the user to the user terminal 200 every predetermined time interval or by a request of the user terminal.

Also, the user terminal 200 is shown in FIG. 19 as receiving the living information LI1, LI2, and LI3, but the present disclosure is limited thereto. The sleep management server SV may receive the living information LI1, LI2, and LI3.

The sleep management system 1 obtains sleep advice for the user (1350).

The sleep advice may include common sense about sleep and advice of a sleep specialist or a metabolic specialist, and may change depending on the type of sleep of the user. For example, the type of sleep of the user is divided into a plurality of sleep type groups, and appropriate sleep advice may be provided for each of the sleep type groups.

Also, the sleep advice may change depending on the living information LI1, LI2, and LI3 of the user. For example, when the user watches a broadcast program even after it's past his or her bedtime, advice indicating that watching a broadcast program after bedtime may disturb deep sleep may be provided to the user. Also, when the user sets an indoor temperature to be high before sleep, advice indicating that a low indoor temperature is helpful for deep sleep may be provided to the user. Also, when the user drinks cold water before sleep, advice indicating that drinking a warm drink before sleep is helpful for deep sleep may be provided to the user.

In detail, the user terminal 200 of the sleep management system 1 may obtain sleep advice on the basis of the sleep information SI of the user and the living information of the user. The sleep advice may be prestored in the storage 240 of the user terminal 200, or may be received from the sleep management server SV.

Also, the sleep management server SV of the sleep management system 1 may generate sleep advice on the basis of the sleep information SI of the user and the living information of the user.

The sleep management system 1 transmits pieces of sleep advice SA1, SA2, and SA3 for the user to the electronic devices 300, 400, and 500 (1360).

As shown in FIG. 20, the user terminal 200 of the sleep management system 1 may transmit the pieces of sleep advice SA1, SA2, and SA3 to the electronic devices 300, 400, and 500, respectively.

For example, the user terminal 200 may transmit sleep advice SA1 associated with watching a broadcast program to the image display apparatus 200. In detail, when the user watches a broadcast program even after his or her bedtime, the user terminal 200 may transmit sleep advice indicating that watching a broadcast program after bedtime may disturb deep sleep to the image display apparatus 300, and the image display apparatus 300 may display the sleep advice.

Also, the user terminal 200 may transmit sleep advice SA2 associated with an indoor temperature during sleep to the air conditioner 400. In detail, when a user turns off the air conditioner 400 or sets the indoor temperature to be high before sleep, the user terminal 200 may transmit sleep advice indicating that a low indoor temperature is helpful for sleep or sleep advice indicating that a sleep mode for deep sleep is provided to the air conditioner 400, and the air conditioner 400 may display the sleep advice.

Also, the user terminal 200 may transmit sleep advice associated with food intake to the refrigerator 500. In detail, when the user opens the door of the refrigerator 500 before sleep, the user terminal 200 may transmit sleep advice indicating that a warm drink before sleep is helpful for sleep to the refrigerator 500, and the refrigerator 500 may display the sleep advice. Also, when the total sleep time of the user is less than an average sleep time, the user terminal 200 may transmit sleep advice that recommends eating food helpful for deep sleep to the refrigerator 400, and the refrigerator 400 may display the sleep advice.

Also, the user terminal 200 may transmit sleep advice associated with outdoor activity of the user to a wearable device. In detail, when the user's outdoor activity is not sufficient, the user terminal 200 may transmit sleep advice indicating that simple exercise is helpful for sleep to the wearable device, and the wearable device may display the sleep advice. Also, when the user's outdoor activity is excessive, the user terminal 200 may provide sleep advice that recommends increasing a sleep time through the wearable device.

Also, the user terminal 200 may directly display sleep advice associated with the use of the user terminal 200. In detail, when the user uses the user terminal 200 in bed for a certain time or longer, the user terminal 200 may provide sleep advice indicating that bright light before sleep may disturb sleep. Also, when the user enters a schedule for an overseas trip or an overseas business trip or purchases an overseas airplane ticket using the user terminal 200, the user terminal 200 may provide sleep advice that recommends changing a sleep time for jet lag.

An example in which the user terminal 200 transmits the pieces of sleep advice SA1, SA2, and SA3 to the plurality of electronic devices 300, 400, and 500 has been described, but the present disclosure is not limited thereto. For example, the sleep management server SV may transmit the pieces of sleep advice SA1, SA2, and SA3 to the plurality of electronic devices 300, 400, and 500.

Also, the sleep management system 1 may transmit the sleep information SI to the plurality of electronic devices 300, 400, and 500, and each of the electronic devices 300, 400, and 500 may directly provide sleep advice to the sleep management system 1.

As described above, the sleep management system 1 may collect activity information of the user from the plurality of electronic devices 300, 400, and 500 and may transmit sleep advice to each of the electronic devices 300, 400, and 500 on the basis of the collected activity information. Also, each of the electronic devices 300, 400, and 500 may provide sleep advice appropriate for the sleep information and the living information of the user to the user.

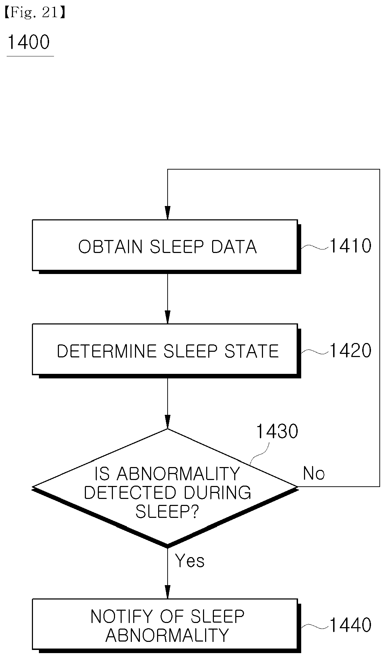

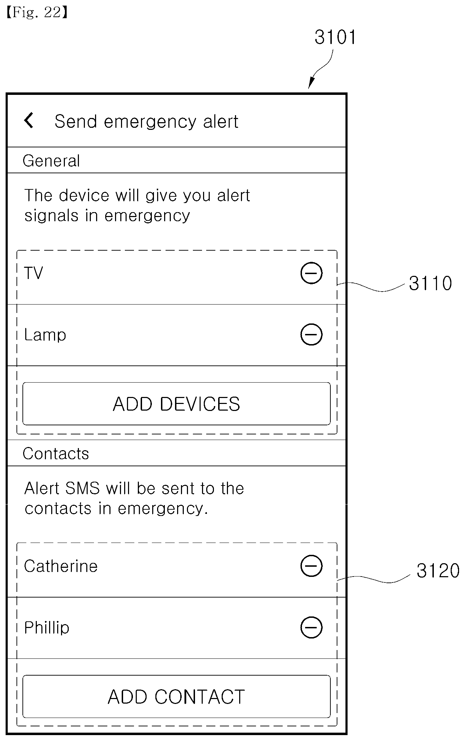

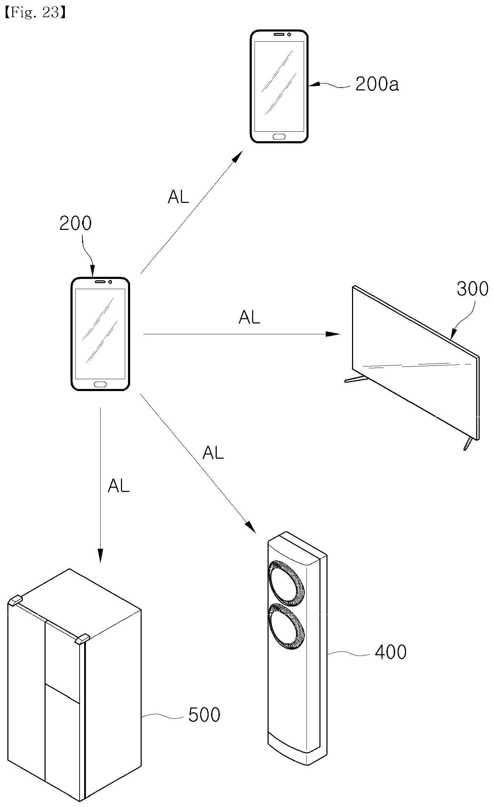

FIG. 21 shows an example of a sleep abnormality notification operation of a sleep management system according to an embodiment. FIG. 22 shows an example of a screen in which a sleep management system obtains a setting of whether to perform notification of an abnormality according to an embodiment. Also, FIG. 23 shows an example in which a sleep management system transmits a sleep abnormality notification to an electronic device according to an embodiment.

A sleep abnormality notification operation 1400 of the sleep management system 1 will be described with reference to FIGS. 21, 22, and 23.

The sleep management system 1 obtains sleep data SD of a user (1410).

A sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep management system 1 determines a sleep state SS of the user (1420).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

Also, the sleep management server SV may determine the sleep state SS of the user on the basis of the sleep data SD received from the user terminal 200.

The sleep management system 1 determines an abnormality during sleep of the user (1430).

The user terminal 200 or the sleep management server SV of the sleep management system 1 may determine a sleep abnormality of the user on the basis of the sleep data SD and the sleep state SS of the user.

For example, when a difference between the user's heart rate and an average heart rate is greater than or equal to 30% of the average heart rate, the user's heart rate is outside a normal heart rate range, or the user's heart rate has an irregular pattern, the user terminal 200 or the sleep management server SV may determine that the user's heart rate is abnormal. In particular, when the user has a circulatory disease, a criterion for his or her heart rate abnormality may change.

Also, when the user's respiration is not detected for a reference time or longer or a respiration pattern of the user is irregular, the user terminal 200 or the sleep management server SV may determine a respiratory disturbance during the user's sleep. In particular, when the user has a respiratory disease, a criterion for a respiratory abnormality may change.

Also, when a sleep score of the user is significantly lower than an average sleep score, the number of times the user wakes up while sleeping is significantly large, or when the a wake-up time of the user is irregular, the user terminal 200 or the sleep management server SV may determine that the user has a sleep disorder. In particular, when the user has a sleep-related disease such as somnambulism, a criterion for a sleep disorder may change.

When no abnormality is detected during the user's sleep (no in 1430), the sleep management system 1 repeats the acquisition of sleep data (1410), the determination of a sleep state (1420), and the detection of an abnormality during sleep (1430).

Also, when an abnormality is detected during the user's sleep (yes in 1430), the sleep management system 1 transmits a sleep abnormality message to the user to a sharer or a home appliance that is set by the user (1440).

In this case, the user may preset the sharer or the home appliance to which the sleep abnormality message is to be transmitted.

For example, the user may set the sharer or the home appliance to which the sleep abnormality message is to be transmitted through an abnormality notification setting screen 3101 shown in FIG. 22.

The abnormality notification setting screen 3101 may include a home appliance setting region 3110 for setting the home appliance to which the sleep abnormality message is to be transmitted and a sharer setting region 3120 for setting the sharer to which the sleep abnormality message is to be transmitted.

The home appliance setting region 3110 may display the home appliance to which the sleep abnormality message is to be transmitted by the user's setting, and may display an addition button region for adding a home appliance.

Also, the sharer setting region 3120 may display the sharer to which the sleep abnormality message is to be transmitted by the user's setting, and may display an addition button region for adding a sharer.

The user terminal 200 or the sleep management server SV may transmit the sleep abnormality message to the sharer and the home appliance that is set by the user through various methods.

For example, as shown in FIG. 23, the user terminal 200 may transmit the sleep abnormality message of the user to a user terminal 200a of the sharer.

In detail, the user terminal 200 or the sleep management server SV may transmit a message indicating a sleep disorder of the user to the sharer according to identification information of the sharer or identification information of the user terminal of the sharer that is input by the user.

For example, when an ID of a sleep management application of the sharer is input, the user terminal 200 may transmit the sleep abnormality message of the user through the sleep management server SV by using an account of the sleep management application of the sharer.

Also, when an e-mail address of the sharer is input, the user terminal 200 may transmit the sleep abnormality message of the user by using the input e-mail address of the sharer.

Also, when a user terminal number of the sharer is input, the user terminal 200 may transmit the sleep abnormality message of the user through a text message by using the input number.

Also, when a sharer ID of a social network service is input, the user terminal 200 may post the sleep abnormality message by using a sharer account of a social network service application.

When the name of the sharer is input, the user terminal 200 may search a contact list to obtain the e-mail address of the sharer or the user terminal number of the sharer. Also, the user terminal 200 may transmit the sleep abnormality message of the user by using the e-mail address of the sharer or may transmit the sleep abnormality message of the user to the user terminal of the sharer through a text message.

When the sleep abnormality message is received, the user terminal 200a of the sharer may display the sleep abnormality message of the user.

Also, as shown in FIG. 22, the user terminal 200 may transmit the sleep abnormality message of the user to electronic devices such as the image display apparatus 300, the air conditioner 400, and the refrigerator 500.

When the sleep abnormality message of the user is received, the image display apparatus 300, the air conditioner 400, and the refrigerator 500 may display the sleep abnormality message of the user together with a warning sound.

In addition, the user terminal 200 may capture a sleep image of the user by using a front capture function of a robot cleaner, and may transmit the captured sleep image to the sharer. Also, the user terminal 200 may transmit an operation start command to the image display apparatus 300 to wake the user.

As described above, when a sleep abnormality is detected during the user's sleep, the sleep management system 1 may transmit a sleep abnormality message of the user to the sharer set by the user. As a result, the sharer may recognize the sleep abnormality of the user and take an appropriate action.

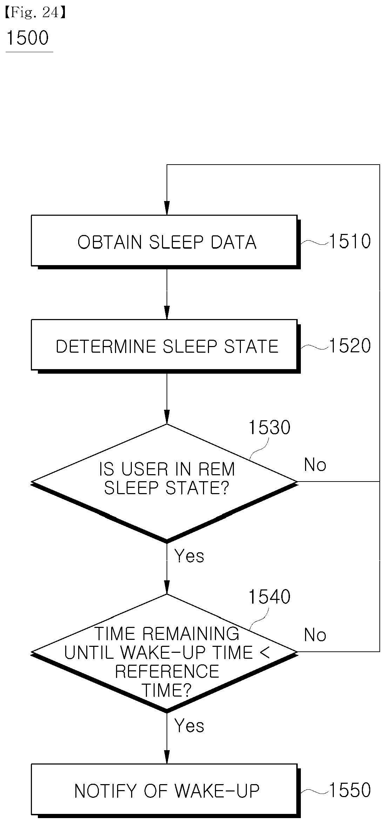

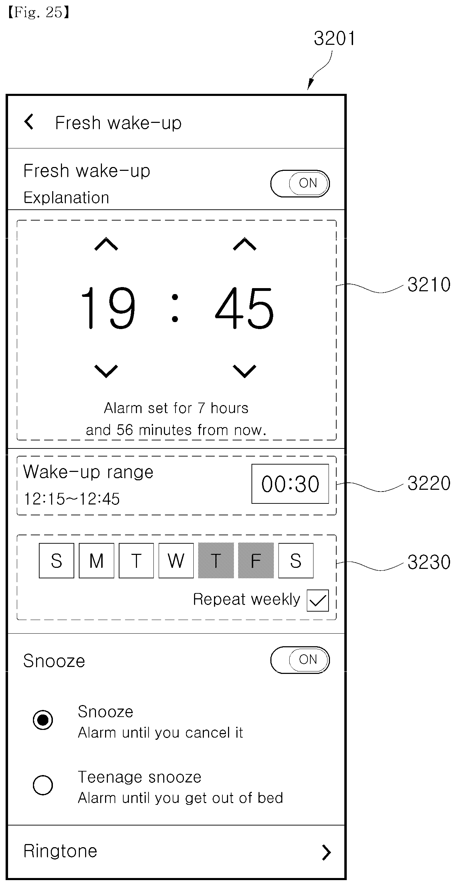

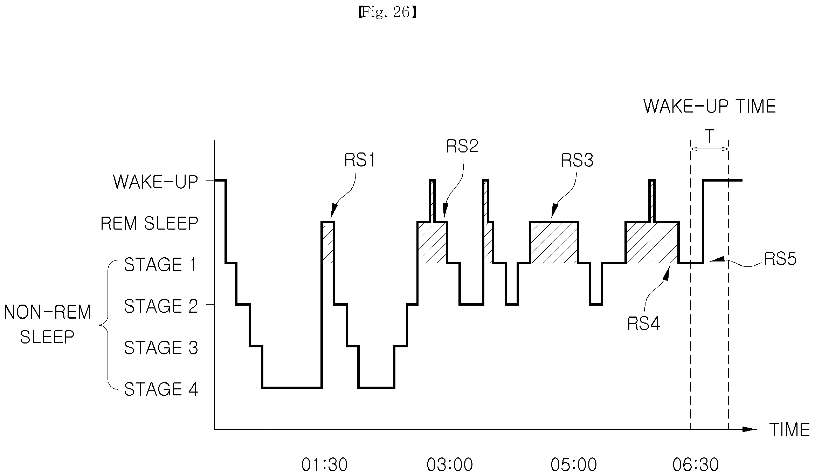

FIG. 24 shows an example of an alarm operation of a sleep management system according to an embodiment. Also, FIG. 25 shows a wake-up time setting screen for an alarm operation of a sleep management system according to an embodiment, and FIG. 26 shows a sleep pattern of a user for illustrating an alarm operation of a sleep management system according to an embodiment.

An alarm operation 1500 of the sleep management system 1 will be described with reference to FIGS. 24, 25, and 26.

The sleep management system 1 obtains sleep data SD of a user (1510).

The sleep data acquisition apparatus 100 of the sleep management system 1 may detect a heart rate, respiration rate, and movement of the user every predetermined time interval and transmit sleep data SD corresponding to the user's heart rate, respiration rate, and movement to the user terminal 200.

Also, when the sleep data SD of the user is received, the user terminal 200 may transmit the sleep data SD to the sleep management server SV.

The sleep management system 1 determines a sleep state SS of the user (1520).

The user terminal 200 of the sleep management system 1 may directly determine the sleep state SS of the user on the basis of the sleep data SD of the user, or may transmit the sleep data SD to the sleep management server SV and receive the sleep state SS from the sleep management server SV.

Also, the sleep management server SV may determine the sleep state SS of the user on the basis of the sleep data SD received from the user terminal 200.

The sleep management system 1 determines whether the sleep state SS of the user is a REM sleep state (1530).

As described above, the user's sleep may be divided into a REM sleep stage and a non-REM sleep stage. Also, a human brain is active in the REM sleep stage, and the REM sleep stage is known as the next stage after a wake-up stage among sleep stages. Also, it is known that people may feel less fatigue and easily wake up when they wake up during REM sleep.

Accordingly, the sleep management system 1 may provide a wake-up alarm to the user during REM sleep so that the user can comfortably wake. To this end, the sleep management system 1 may determine whether the user is in REM sleep.

In detail, the user terminal 200 or the sleep management server SV may determine the sleep state SS, such as whether the user lies on a bed, whether the user falls asleep, in which sleep stage the user is, whether the user wakes up briefly while sleeping, whether the user falls asleep again, whether the user completely wakes up, and whether the user gets out of bed, on the basis of the sleep data SD of the user.

The user terminal 200 or the sleep management server SV may determine whether the user is in REM sleep on the basis of the sleep state SS of the user.

When the user is not in REM sleep (no in 1530), the sleep management system 1 repeats the acquisition of sleep data (1510), the determination of a sleep state (1520), and the determination of whether the user is in REM sleep (1530).

Also, when the user is in REM sleep (yes in 1530), the sleep management system 1 determines whether a time remaining until a wake-up time set by the user is within an alarm time range (e.g., 30 minutes) (1540).

Here, the wake-up time may be preset by the user.

For example, the user may set the wake-up time through a wake-up time setting screen 3201, as shown in FIG. 25.

A wake-up time setting region 3210 for setting the wake-up time of the user, an alarm time setting region 3220 for inputting a range in which an alarm is output on the basis of the set wake-up time, and an alarm repetition setting region 3230 for setting repetition of the alarm may be displayed in the wake-up time setting screen 3201.

The user may input a desired wake-up time through the wake-up time setting region 3210.

Also, the user may select a time range in which the wake-up alarm is output through the alarm time setting region 3220.

As shown in FIG. 26, a plurality of REM sleep stages RS1, RS2, RS3, RS4, and RS5 occur during the user's sleep. Since human sleep repeats light sleep and deep sleep at regular intervals, the plurality of REM sleep stages RS1, RS2, RS3, RS4, and RS5 may be detected during human sleep.

In order to induce the user to freshly wake-up and provide a sufficient sleep time to the user, the sleep management system 1 may provide an alarm to the user during a REM sleep stage closet to the wake-up time set by the user while the user is in REM sleep.

Here, the alarm time setting region 3220 indicates a time range in which the sleep management system 1 can provide an alarm to the user.

Also, the user may select a date at which the wake-up alarm will be provided on a weekly basis through the alarm repetition setting region 3230. For example, as shown in FIG. 25, when Thursday (T) and Friday (F) are selected, the sleep management system 1 may provide an alarm to the user on Thursday and Friday during a week.

Also, the user terminal 200 or the sleep management server SV may compare the current time with the wake-up time set by the user and may determine whether a difference between the current time and the wake-up time is within an alarm time range set by the user. In other words, the user terminal 200 or the sleep management server SV may determine whether the current time approaches the wake-up time set by the user.

When a time remaining until the wake-up time is not within the alarm time range (no in 1540), the sleep management system 1 repeats the acquisition of sleep data (1510), the determination of a sleep state (1520), and the determination of whether the user is in REM sleep (1530).

For example, as shown in FIG. 26, the user may experience first REM sleep RS1, second REM sleep RS2, third REM sleep RS3, fourth REM sleep RS4, and fifth REM sleep RS4 while sleeping.

In this case, REM sleep times of the first REM sleep RS1, the second REM sleep RS2, the third REM sleep RS3, and the fourth REM sleep RS4 are not within the reference time from the wake-up time, and thus the sleep management system 1 does not provide a wake-up notification to the user.

Also, when the time remaining from the wake-up time is within the alarm time range (yes in 1540), the sleep management system 1 provides the wake-up alarm to the user (1550).

When the user is in REM sleep and the time remaining until the wake-up time is less than the reference time, the user may easily wake up from sleep. Accordingly, the sleep management system 1 may provide the wake-up alarm to the user.

For example, as shown in FIG. 26, the REM sleep time of the fifth REM sleep RS5 is within the reference time from the wake-up time, and thus the sleep management system 1 may provide the wake-up alarm to the user.

In detail, the user terminal 200 may output an alarm sound preset by the user.

As described above, in order to induce the user to freshly wake-up, the sleep management system 1 may provide a wake-up alarm to the user when the user's REM sleep is detected.

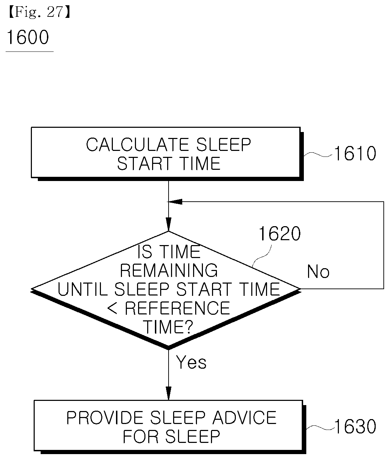

FIG. 27 shows an example of a sleep inducing operation of a sleep management system according to an embodiment.

A sleep inducing operation 1600 of the sleep management system 1 will be described with reference to FIG. 27.

The sleep management system 1 calculates a sleep start time of a user (1610).

The user terminal 200 or the sleep management server SV may calculate the sleep start time of the user on the basis of a wake-up time set by the user and an average sleep time of the user. For example, the user terminal 200 or the sleep management server SV may calculate a recommended sleep start time of the user by subtracting the average sleep time from the wake-up time set by the user.

The sleep management system 1 determines whether a time remaining until the recommended sleep start time of the user is less than a reference time (1620).

In order to enable the user to sleep during at least the average sleep time, the sleep management system 1 may determine whether the time remaining until the sleep start time of the user is less than the reference time. Here, the reference time may be determined as an average time it takes the user to fall asleep after lying on a bed.

When the time remaining until the recommended sleep start time of the user is not less than the reference time (no in 1620), the sleep management system 1 may wait.

Also, when the time remaining until the recommended sleep start time of the user is less than the reference time (no in 1620), the sleep management system 1 may provide sleep advice for comfortable sleep to the user (1630).

The user terminal 200 of the sleep management system 1 may generate a vibration to call attention of the user and display the sleep advice to the user along with the vibration.

Also, the user terminal 200 may transmit the sleep advice for comfortable sleep to the electronic devices 300, 400, and 500, such as the image display apparatus 300, the air conditioner 400, and the refrigerator 500. As a result, the electronic devices 300, 400, and 500, such as the image display apparatus 300, the air conditioner 400, and the refrigerator 500, may display sleep advice for fresh sleep to the user.

As described above, in order to enable the user to comfortably sleep, the sleep management system 1 may recommend a sleep start time to the user and may provide sleep advice for comfortable sleep to the user.

While an embodiment of the present disclosure has been shown and described, the disclosure is not limited to the above-described specific embodiment. Also, it should be understood by those skilled in the art that various changes may be made therein without departing from the spirit and scope of the disclosure as defined by the appended claims, and also the various changes are not to be understood as departing from the spirit and scope of the present disclosure.

## Claims

1. A sleep management method comprising:
obtaining sleep data of a user;
obtaining sleep information of the user on the basis of the sleep data of the user;
identifying a user sleep type group to which a sleep type of the user belongs among a plurality of sleep type groups on the basis of the sleep information of the user; and
displaying sleep advice corresponding to the user sleep type group.

2. The sleep management method of claim 1, further comprising calculating a sleep score of the user on the basis of the sleep information of the user.

3. The sleep management method of claim 2, further comprising displaying both the sleep score of the user and an average sleep score of an age group in which the user is included.

4. The sleep management method of claim 1, further comprising displaying the sleep information of the user.

5. The sleep management method of claim 1, further comprising receiving physical information of the user from the user,
wherein the physical information includes at least one of a gender, height, and weight of the user.

6. The sleep management method of claim 5, further comprising displaying sleep advice corresponding to the physical information of the user.

7. The sleep management method of claim 1, further comprising receiving activity information of the user from the user,
wherein the activity information includes at least one of whether the user drinks, whether the user takes a stimulant, and whether the user exercises.

8. The sleep management method of claim 7, further comprising displaying sleep advice corresponding to the activity information of the user.

9. The sleep management method of claim 1, further comprising receiving living information of the user from at least one electronic device,
wherein the living information includes at least one of a user input received by the electronic device from the user and detection data detected by the electronic device from the user.

10. The sleep management method of claim 9, further comprising displaying sleep advice corresponding to the living information of the user.

11. The sleep management method of claim 9, further comprising transmitting sleep advice corresponding to the living information of the user to the at least one electronic device.

12. The sleep management method of claim 1, further comprising sharing the sleep information and the sleep advice with a sharer set by the user.

13. The sleep management method of claim 12, wherein the sharing of the sleep information and the sleep advice comprises:
receiving at least one of identification information of the sharer and identification information of a user terminal of the sharer; and
transmitting the sleep information and the sleep advice to the user on the basis of the identification information of the sharer and the identification information of the user terminal of the sharer.

14. The sleep management method of claim 12, wherein the sharing of the sleep information and the sleep advice with a sharer comprises:
receiving the sleep information and a sharing time of the sleep information and the sleep advice from the user; and
transmitting the sleep information and the sleep advice to the sharer at the sharing time.

15. The sleep management method of claim 1, further comprising:
identifying a sleep abnormality of the user on the basis of the sleep data of the user and the sleep state of the user; and
transmitting a sleep abnormality message of the user in response to identifying of the sleep abnormality of the user.
